# EUROPEAN PATENT APPLICATION

(11) **EP 2 508 510 A1**
(43) Date of publication of application: **10.10.2012**
(21) Application number: 11382101.1
(22) Date of filing: 06.04.2011
(51) Int. Cl.: C07D 207/34, C07D 405/04, C07D 409/04, A61K 31/40, A61K 31/4025, A61P 35/00

(54) **Hydroxyphenyl pyrrole compounds containing an hydroxamic acid as hdac inhibitors and medicinal applications thereof**

(71) Applicant: Ikerchem, S.L., 20009 San Sebastian - Donostia Guipuzcoa (ES); Universidad Del Pais Vasco Euskal Herriko Unibertsitatea, 48940 Leioa (Vizcaya) (ES)
(72) Inventor: Cossio Mora, Fernando Pedro, E-20018, San Sesbastian (ES); Vara Salarzar, Yosu Ion, Donostia, Giupuzcoa E-20009, San Sebastián (ES); San Sebastian Larzabal, Eider, E-20009, San Sebastián - Donostia, Guipúzcoa (ES); Otaegui Ansa, M Dorleta, E-20009, San Sebastián - Donostia, Guipúzcoa (ES); Masdeu Margalef, M Maria del Carmen, E-20009, San Sebastián - Donostia, Guipúzcoa (ES); Aldaba Arevalo, Mr Eneko, E-20009, San Sebastián - Donostia, Guipúzcoa (ES); Aginagalde Unanue, M Maialen, E-20018, San Sesbastian (ES); Villafruela Caneva, Mr Sergio, E-20009, San Sebastián - Donostia, Guipúzcoa (ES); Alcala Caffrarena, M Maria Remedios, E-20009, San Sebastián - Donostia, Guipúzcoa (ES); Zubia Olascoaga, M Aizepa, E-20009, San Sebastián - Donostia, Guipúzcoa (ES)
(74) Representative: ABG Patentes, S.L.

(57) **Abstract**

The present invention refers to compounds derived from hydroxyphenyl 1H-pyrrole rings, which have the following formula (I): 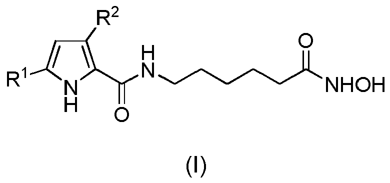 as well as to the procedure for their preparation, pharmaceutical compositions comprising the same and the use thereof for the treatment and/or prevention of a condition mediated by histone deacetylase such as cancer, hematological malignancies, autoimmune diseases, inflammatory diseases, diseases of the central nervous system (CNS) such as neurodegenerative diseases and psychiatric disorders, cardiovascular diseases, endocrine and metabolic disorders, by inhibiting histone deacetylases and the therapeutic use of biological processes related to the mentioned inhibition.

## Description

### Field of the invention

The present invention relates to compounds, compositions, formulations, methods of use and manufacture of novel hydroxyphenyl pyrrole derivatives as inhibitors of histone deacetylase enzymes and therapeutic agents for preventing and/or treating diseases associated with histone and non-histone protein hypoacetylation such as proliferative diseases, cancer, hematological malignancy, neurological and psychiatric disorders, diseases of the central nervous system, endocrine and metabolic disorders and inflammatory and immune conditions.

### Background of the invention

### Technical background

Protein function is often regulated by posttranslational chemical modifications, such as the enzymatic addition or removal of acetyl groups at specific lysine residues. The reversible acetylation of lysine residues in histone and non-histone proteins is the result of a dynamic equilibrium between competing histone deacetylases (HDACs) and histone acetyltransferases (HATs). HDACs catalyze the removal of the acetyl modification on lysine residues of the core nucleosomal histones H2A, H2B, H3 and H4, and other non-histone proteins such as nuclear receptors p53, E2F, c-Myc, nuclear factor κB, hypoxia-inducible factor 1 alpha (HIF-1α), signal transducer and activator of transcription 3 (STAT3), as well as α-tubulin and heat shock protein 90 (Hsp90) (cf. M. A. Glozak et al. Gene 2005, 363, 15) and the transcription factor Bcl-6 (cf. O. R. Bereshchenko, W. Gu, R. Dalla-Favera Nat Genet 2002, 32, 606).

HDAC enzymes are divided into 4 different classes: Class I (HDACs 1, 2, 3 and 8), class II (HDACs 4, 5, 6, 7, 9 and 10), class III (SIRT 1-7) and class IV (HDAC 11). Classes I, II and IV are zinc-dependent enzymes, whereas class III HDAC enzymes (also known as sirtuins) are structurally unrelated and use NAD⁺ as a cofactor for their catalytic activity instead of zinc, being generally not inhibited by class I and II inhibitors (cf. C. M. Grozinger, S. L. Schreiber Chem Biol. 2002, 9, 3; A. J. M. de Ruijter et al. Biochem. J. 2003, 370, 737).

HDACs play important roles in multiple physiological processes, and are also aberrantly expressed or show disregulated activity in multiple pathological conditions, such as cancer, neurological and psychiatric disorders, endocrine and metabolic disorders and inflammatory and immune conditions. Aberrant gene expression that results in functional inactivation of HAT activity or overexpression of HDACs can provoke histone target deacetylation, which leads to tumor development (cf. T. Kouzarides Curr. Opin. Genet. Dev. 1999, 9, 40). In addition, HAT/HDACs are able to modify the acetylation pattern of numerous other nonhistone targets, such as proteins involved in cellular proliferation, migration, DNA repair, cell death, angiogenesis, inflammation, and the immune response (cf. O. P. Kallioniemi et al. Int J Cancer 1991, 49, 650) all of which are key for tumor formation and progression. Correspondingly, HDAC inhibitors are important therapeutic anticancer agents that function via multiple mechanisms, such as induction of cell-cycle arrest, apoptosis, autophagic cell death, mitotic cell death, reactive oxygen species, inhibiting angiogenesis (cf. O. P. Kallioniemi et al. *op*. *cit*.) and improving NK cell-mediated tumor immunity (cf. S. Weng et al. J Clin Oncol 2009, 27(15S), e14587). These diverse effects on cancer cells make HDAC inhibitors attractive both for monotherapy and for combination therapy in clinical cancer conditions (cf. P. A. Marks et al. Nat. Rev. Cancer 2001, 1, 194; J. E. Bolden et al. Nat Rev Drug Discov. 2006, 5, 769; P. Gallinari et al. Cell Res. 2007, 17, 195; K. B. Glaser Biochem. Pharmacol. 2007, 74, 659; L. N. Pan et al. Cell. Mol. Immunol. 2007, 4, 337; M. Haberland et al. Nat. Rev. Genet. 2009, 10, 32; Y. Zhang et al. Curr. Med. Chem. 2008, 15, 2840; S. Ropero, M. Esteller Mol. Oncol. 2007, 1, 19).

Many neurodegenerative disorders exhibit characteristic symptoms of learning and memory impairment which ultimately lead to a decline in quality of life. It was found that many transcriptional changes occur during the learning process and that these processes may be downregulated in neurodegenerative disease states (cf. C. G. Vecsey et al. J Neurosci 2007, 27, 6128; A. Fontán-Lozano et al. Mol Cell Neurosci 2008, 39, 193). Treatment with various HDAC inhibitors can correct these deficiencies and has emerged as a promising new strategy for therapeutic intervention in neurodegenerative disease. Currently, a variety of HDAC inhibitors are being investigated at both the research as well as the clinical level for a host of neurodegenerative disease states including Rubinstein-Taybi syndrome, Rett syndrome, Friedreich's ataxia, HD, multiple sclerosis, anxiety, and schizophrenia (cf. T. Abel, R. S. Zukin Curr Opin Pharmacol 2008, 8, 57; A. Guidotti et al. Trends Pharmacol Sci 2009, 30, 55; A. G. Kazantsev, L. M. Thompson Nat Rev Drug Discov 2008, 7, 854).

HDAC play critical roles in modulation of chromatin architecture, and govern the expression of oncogenes, tumor suppressor genes and inflammatory genes (cf. A. J. M. Ruijter et al. *op. cit*.). There is now emerging evidence that HDAC inhibitors could have utility as therapeutics in the treatment of chronic immune and inflammatory disorders such as rheumatoid arthritis, psoriasis, inflammatory bowel disease (IBD), multiple sclerosis and systemic lupus erythematosus (cf. L. Bäckdahl et al. Int J Biochem Cell Biol. 2009, 41, 176; Q. Y. Choo et al. Curr Pharm Des 2008, 14, 803). There is now evidence for an anti-inflammatory effect of histone hyperacetylation. In fact, it is known that HDAC inhibition is associated with a significant suppression of proinflammatory cytokines; therefore, it was proposed to comprise an anti-inflammatory effect (cf. R. Glauben, B. Siegmund Methods Mol Biol. 2009, 512, 365). In addition, HDACs have been observed to activate nuclear factor kappa B (NFkB), a key mediator of inflammatory cascades (cf. M.W. Lawless et al. Endocr Metab Immune Disord Drug Targets 2009, 9, 84). Also, recent studies illustrate the role of HDACs in maintaining TH1-like and TH2-like immunity in human T cells (cf. R. C. Su et al. J Allergy Clin Immunol 2008, 121, 57).

As for the relationship between HDACs and endocrine and metabolic disorders, *in vivo* relevance has been elucidated by examining histone acetylation patterns in onocytes from diabetic patients (cf. M. Guha et al. J Biol Chem 2000, 275, 17728). These results demonstrate high glucose condition mimicking diabetes can have an effect on *in vivo* chromatin remodelling in both cell culture and in patients by increasing acetylation of specific lysine residue from histones 3 and 4 and by activation of transcription factor NF-κB and HAT at the promoters of inflammatory genes.

Several families of HDAC inhibitors (HDACis) have been designed, whose general structures can be found in different reviews (cf. A. Villar-Garea, M. Esteller Int. J. Cancer 2004, 112, 171; T. A. Miller et al. J. Med. Chem. 2003, 46, 5097; T. Suzuki, N. Miyata Curr. Med. Chem. 2005, 12, 2867; M. Paris et al. J. Med. Chem. 2008, 51, 1505). The general structure of the most active inhibitors consists of a cyclic structure, a spacer and a chelating group capable of binding to the Zn (II) cation of the active center of the different HDAC isoforms that belong to the class I, class II and class IV.

To date, the US FDA has granted the approval of two HDAC inhibitors, which have been marketed for the treatment of cutaneous T cell lymphoma (CTCL): Vorinostat (SAHA, trade name Zolinza) and Romidepsin (also called depsipeptide or FK228, trade name Istodax). Several other structurally diverse HDAC inhibitors have entered clinical trials, such as Mocetinostat (MGCD0103), Panobinostat (LBH589), Belinostat (PXD101), PCI-24781, Entinostat (MS-275), AR-42, 4SC-201, DAC60, R306465 (JNJ16241199), CHR-3996, Pyroxamide, Chidamide (HBI-8000), CUDC101 or SB939, either in combination therapy or as single agents, for the treatment of solid and hematological cancers (cf. R. L. Piekarz, S. E. Bates Clin. Cancer Res. 2009, 15, 3918; H. Prince et al. Clin. Cancer Res. 2009, 15, 3958). All these compounds are HDAC pan-inhibitors, or class selective HDAC inhibitors, but none of them show any isoform specificity or selectivity.

Increasing evidence suggests that selectivity in the inhibition of different HDAC isoforms is of therapeutic interest (cf. K. V. Butler, A. P. Kozikowski Curr. Pharm. Des 2008, 14, 505; T. C. Karagiannis, A. El-Osta Leukemia 2007, 21, 61). In general, selective HDAC inhibitors have the potential for less off-target effects and, as a consequence, less potential toxicity. Therefore, selective HDAC inhibitors, which affect either a single HDAC isoform (isoform selective HDACi) or several isoforms within a single class (class selective HDACi) would probably provide a chemotherapy with a more favourable toxicological profile compared to pan-inhibitors and probably higher chances to succeed in clinical trials and reach the market.

Different publications highlight the pathophysiological importance of HDAC6. HDAC6 participates in the deacetylation of non-histone proteins such as α-tubulin (cf. C. Hubbert et al. Nature 2002, 417, 455; A. Matsuyama et al. EMBO J. 2002, 21, 6820), cortactin, β-catenin, the chaperone Hsp90, and the redox regulatory proteins peroxiredoxin (Prx) I and Prx II (cf. C. Boyault et al. Oncogene 2007, 26, 5468; P. Matthias et al. Cell Cycle 2008, 7, 7; Y. Li et al. J Biol. Chem. 2008, 283, 12686; R. B. Parmigiani et al. Proc. Natl Acad. Sci USA 2008, 105, 9633). Thus, HDAC6 mediates a wide range of cellular functions including microtubule-dependent trafficking and signaling, membrane remodeling and chemotactic motility, involvement in control of cellular adhesion, ubiquitin level sensing, regulation of chaperone levels and activity, and responses to oxidative stress. Also, HDAC6 regulates many important biological processes involved in cancer progression, inflammation and neurodegenerative diseases, including cell migration, tumor cell proliferation and survival, tumor angiogenesis, microtubule stability and function, molecular chaperon activity and the degradation of misfolded proteins (cf. A. Valenzuela-Fernández et al. Trends Cell Biol 2008, 18, 291).

Diseases in which selective HDAC6 inhibition could have a potential benefit include **cancer** (cf. T. Simms-Waldrip et al. Mol Genet Metab 2008, 94, 283; A. Rodriguez- Gonzalez et al. Cancer Res. 2008, 68, 2557), specifically: multiple myeloma (cf. T. Hideshima et al. Proc. Natl Acad Sci. USA 2005, 102, 8567); lung cancer (cf. K. Kamemura et al. Biochem. Biophys. Res. Commun. 2008, 374, 84); ovarian cancer (cf. M. Bazzaro et al. Clin. Cancer Res. 2008, 14, 7340); breast cancer (cf. Y. S. Lee et al. Cancer Res. 2008, 68, 7561); prostate cancer (cf. B. Mellado et al. Clin. Transl. Oncol. 2009, 11, 5); pancreatic cancer (cf. S. T. Nawrocki et al. Cancer Res. 2006, 66, 3773); renal cancer (cf. T. L. Cha et al. Clin. Cancer Res. 2009, 15, 840); and leukemias such as acute myeloid leukemia (AML) (cf. W. Fiskus et al. Blood 2008, 112, 2896) and acute lymphoblastic leukemia (ALL) (cf. Rodriguez-Gonzalez et al. Blood 2008, 112(11), Abstract 1923). Inhibition of HDAC6 may also have a role in **cardiovascular disease**, i.e. cardiovascular stress, including pressure overload, chronic ischemia, and infarction-reperfusion injury (cf. P. Tannous et al. Circulation 2008, 117, 3070); **bacterial infection**, including those caused by uropathogenic Escherichia coli (cf. B. K. Dhakal, M. A. Mulve J. Biol. Chem. 2009, 284, 446); **neurological diseases** caused by accumulation of intracellular protein aggregates such as Huntington's disease (cf. A. G. Kazantsev, L. M. Thompson *op. cit*.; J. P. Dompierre et al. J. Neurosci. 2007, 27, 3571; A. P. Kozikowski et al. J. Med. Chem. 2007, 50, 3054) or **central nervous system** trauma caused by tissue injury, oxidative-stress induced neuronal or axomal degeneration (cf. M. A. Rivieccio et al. Proc. Natl. Acad. Sci. USA 2009, 106, 19599); and **inflammation**, including reduction of proinflammatory cytokine IL-1β (cf. S. Carta et al. Blood 2006, 108, 1618), increased expression of the FOXP3 transcription factor, which induces immunosuppressive function of regulatory T-cells resulting in benefits in chronic diseases such as rheumatoid arthritis, psoriasis, multiple sclerosis, lupus and organ transplant rejection (cf. L. Wang et al. Nat. Rev. Drug Discov. 2009, 8, 969).

Thus, HDAC6 is a potential therapeutic target that regulates multiple functions. Nevertheless, there remains a need in the art for new, well-tolerated and efficacious HDAC6 isotype-selective inhibitors which have a potential therapeutic effect in the mentioned pathological conditions.

### State of the Art

As mentioned, HDAC inhibitors are in general known in the art (cf. A. Villar-Garea, M. Esteller *op*. *cit*.; T. A. Miller et al. *op. cit*.; T. Suzuki, N. Miyata *op*. *cit*.; M. Paris et al. *op*. *cit*.), but there is only limited data published regarding selectivity of HDAC6 inhibition.

The first HDAC6-selective inhibitor described was a 1,3-dioxane-containing hydroxamate analog named Tubacin (cf. S. J. Haggarty et al. Proc. Natl. Acad. Sci. USA 2003, 100, 4389), which showed the capability of inducing tubulin hyperacetylation and synergistically augment bortezomib-induced cytotoxicity in multiple myeloma (cf. T. Hideshima et al. *op*. *cit*.).

Tert-Butyl carbamate-containing thiols (cf. T. Suzuki et al. J. Med. Chem. 2006, 49, 4809; Y. Itoh et al. J Med Chem 2007, 50, 5425) show nanomolar activity against HDAC6, as well as 39-fold selectivity over HDAC-1 and 37-fold over HDAC-4. In addition, these compounds impair the growth of estradiol stimulated MCF-7 breast cancer cells and significantly increased the effect of paclitaxel on HCT116 cancer cell growth inhibition (cf. Y. Itoh et al. J. Med. Chem. 2007 *op*. *cit*.).

Phenylalanine-containing hydroxamic acids (cf. S. Schäfer et al Bioorg Med Chem 2008, 16, 2011) and pyridylalanine-containing hydroxamic acids (cf. S. Schäfer et al Chem Med Chem 2009, 4, 283-290) show micromolar potency against HDAC6 and 5- to 25-fold selectivity over HDAC-1 respectively.

Triazolylphenyl-containing hydroxamic acids (cf. Y. Chen et al. J Med Chem 2008, 51, 3437) show low nanomolar potency and 51-fold selectivity over HDAC-1. These compounds are able to inhibit the growth of 5 different pancreatic cancer cell lines (cf. Y. Chen et al. *op*. *cit*.; R. He et al. J Med Chem 2010, 53, 1347).

Phenylisoxazole-containing hydroxamic acids (cf. A.P. Kozikowski et al. J Med Chem 2008, 51, 4370) are the most potent HDAC6 inhibitors described so far, with an IC50 value in the picomolar range and 210-fold selectivity over HDAC-3. However, these compounds are in vivo hydrolyzed to the corresponding aniline, whose potency and selectivity is probably considerably reduced. In addition, anilines are highly toxic.

Trithiocarbonates (cf. F. Dehmel et al. J. Med. Chem. 2008, 51, 3985) show nanomolar potency and 18-fold selectivity over HDAC-1.

Chiral 3,4-dihydroquinoxalin-2-one and piperazine-2,5-dione aryl hydroxamates (cf. D. V. Smil et al. Bioorg Med Chem Lett 2009, 19, 688) also inhibit HDAC6 in the nanomolar range and show 41-fold selectivity over HDAC-2 and 91-fold over HDAC-8. Tricyclic carbazole hydroxamic acids such as Tubastatin (cf. K. V. Butler et al. J Am Chem Soc 2010, 132, 10842) show low nanomolar potency and 1000-fold selectivity over HDAC-1 and 57-fold selectivity over HDAC-8. Tubastatin also shows dose-dependent protection against neuronal cell death in vitro via peroxiredoxin acetylation.

Other patent literature also refers to compounds that inhibit HDAC6, such as Tubacin and related compounds (US 2006/0239909) which are indicated to protein degradation disorders and cancer, dipyridinyl aminoheptanoic acid hydroxamide derivatives (WO 2010/086646) which are indicated to treat cancer and inflammatory diseases, or hydrazide-functionalized cinnamic hydroxamic acids (WO 2011/019393).

However, the use of hydroxyphenylpyrrole derivatives as HDAC6-selective inhibitors has not been described so far.

On the other hand, it is known that pyrrole derivatives may show HDAC inhibitory activity. For example, the synthesis of 3-(4-aroyl-1-methyl-1H-2-pyrrolyl)-N-hydroxy-2-propenamides has been described as inhibitors of HDAC (cf. A. Mai et al. J. Med. Chem. 2004, 47, 1098). In this case, the spacer chain is unsaturated and positions 3 and 5 of the pyrrole ring are not substituted, resulting in a linear molecular geometry. N-Sulfonylpyrrole derivatives (WO 2006/105979) have also been described, the positions 2, 4 and 5 of the pyrrole ring being substituted with hydrogen or lower alkyl at position. However, the mentioned pyrrole derivative compounds are pan-inhibitors or show low selectivity.

WO 2007/074176 describes some substituted pyrrole hydroxamide derivatives as inhibitors of HDAC. This document does not mention the above described different isotypes of the HDAC enzymes and does not describe a potential isoform selectivity of the compounds disclosed therein.

(Aryloxopropenyl)pyrrolyl hydroxamates have been reported as class II selective HDAC inhibitors (cf. A. Mai et al. J Med Chem 2005, 48, 3344). Inhibitory activity was tested using maize HDAC homologue HD2, HD1-A and HD1-B enzymes, showing a inhibitory activity in the micromolar range. When tested against human HDAC isoforms, the reported compound showed inhibitory activity in the micromolar range against isoform HDAC4. Activity against HDAC6 isoform is not reported.

In spite of this background, there is still a need in the art to provide alternative compounds which act as inhibitors of histone deacetylase enzymes (HDAC). Further, it would be highly desirable to find new compounds having a high selectivity in the inhibition of different HDAC enzymes, especially against HDAC6, given the lack of selective inhibitors of this isoform.

### Object of the invention

The inventors of the present invention have surprisingly found a family of structurally distinct pyrrole derivatives which are particularly useful inhibitors of the histone deacetylases (HDAC). The compounds are hydroxamic acids containing a pyrrole ring which is characterized by the substitution at positions 3 or 5 by at least a phenol group.

More particularly, it has now been found that these hydroxyphenyl pyrrole derivatives, which are described in greater detail below, are more potent inhibitors of the histone deacetylases (HDAC) than their related phenyl or methoxyphenyl pyrrole derivatives. Also, they show improved activity against isoform HDAC6. Moreover, they show improved selectivity against isoform HDAC6.

Thus, the present invention has as an object the hydroxyphenyl pyrrole derivatives of general formula (I): 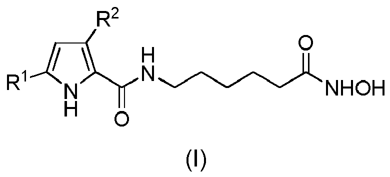 or salts, solvates or prodrugs thereof.

Likewise, another object of the present invention refers to processes for the preparation of a compound of general formula (I), or a salt, solvate or prodrug thereof.

Another aspect of the present invention relates to a compound of general formula (I), or a pharmaceutically acceptable salt, solvate or prodrug thereof, for use as a medicament.

Another aspect of the present invention relates to a compound of general formula (I), or a pharmaceutically acceptable salt, solvate or prodrug thereof, for use as a medicament for the treatment and/or prevention of diseases or disorders responsive or sensitive to the inhibition of histone deacetylases, including without limitation proliferative diseases such as cancer, hematological malignancies, autoimmune diseases, inflammatory diseases, cardiovascular diseases, diseases of the central nervous system (CNS), neurodegenerative diseases, and endocrine and metabolic disorders.

Another aspect of the present invention relates to the use of a compound of general formula (I), or a pharmaceutically acceptable salt, solvate or prodrug thereof, in the preparation of a medicament for the treatment and/or prevention of diseases or disorders responsive or sensitive to the inhibition of histone deacetylases, including without limitation proliferative diseases such as cancer, hematological malignancies, autoimmune diseases, inflammatory diseases, cardiovascular diseases, diseases of the central nervous system (CNS), neurodegenerative diseases, and endocrine and metabolic disorders.

According to another aspect, the present invention is directed to a method of treating and/or preventing diseases or disorders responsive or sensitive to the inhibition of histone deacetylases (including without limitation proliferative diseases such as cancer, hematological malignancies, autoimmune diseases, inflammatory diseases, cardiovascular diseases, diseases of the central nervous system (CNS), neurodegenerative diseases, and endocrine and metabolic disorders), comprising administering to a patient needing such treatment, a therapeutically effective amount of at least one compound of general formula (I) or a pharmaceutically acceptable salt, solvate or prodrug thereof.

According to a particular embodiment, the compounds of general formula (I) are useful for the treatment of various types of cancer by restricting tumor growth or other processes that stop the development of primary or metastatic tumors, through the inhibition of certain histone deacetylases.

Finally, this invention has as an object a pharmaceutical composition comprising at least one compound of general formula (I), or a pharmaceutically acceptable salt, solvate or prodrug thereof, and at least one pharmaceutically acceptable excipient.

These aspects and preferred embodiments thereof are additionally also defined in the claims.

### Detailed description of the invention

First, the present invention provides compounds derived from hydroxyphenyl 1*H*-pyrrole rings that have the following formula (I): 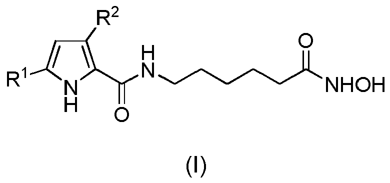 wherein R¹ and R² are independently selected between an optionally substituted C₆-C₁₀ aryl and an optionally substituted 3- to 10-membered heteroaryl group, being at least one of R¹ and R² a phenol group;
or a salt, solvate or prodrug thereof.

In a preferred embodiment, the compounds of general formula (I) are selected from:
[1] *N*-[6-(hydroxyamino)-6-oxohexyl]-3-(4-hydroxyphenyl)-5-phenyl-1*H*-pyrrole-2-carboxamide, with the following structural formula: 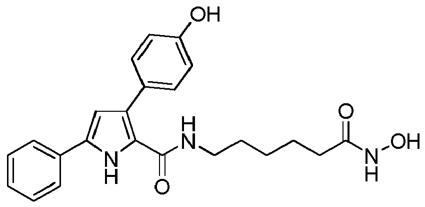
[2] *N*-[6-(hydroxyamino)-6-oxohexyl]-5-(4-hydroxyphenyl)-3-phenyl-1*H*-pyrrole-2-carboxamide, with the following structural formula: 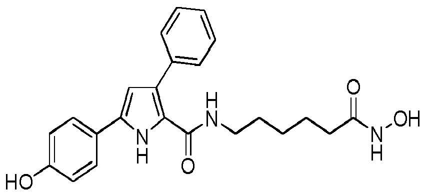
[3] *N*-[5-(Hydroxycarbamoyl)pentyl]-3-(4-hydroxyphenyl)-5-(3-thienyl)-1*H*-pyrrole-2-carboxamide, with the following structural formula: 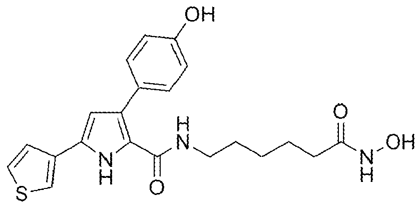
[4] 5-(3-Furyl)-*N*-[5-(Hydroxycarbamoyl)pentyl]-3-(4-hydroxyphenyl)-1*H*-pyrrole-2-carboxamide, with the following structural formula: 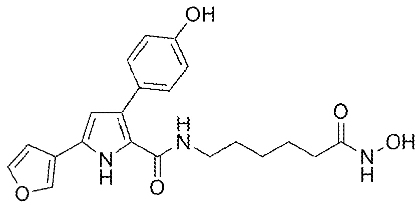
[5] 3-(3-Furyl)-*N*-[5-(hydroxycarbamoyl)pentyl]-5-(4-hydroxyphenyl)-1*H*-pyrrole-2-carboxamide, with the following structural formula: 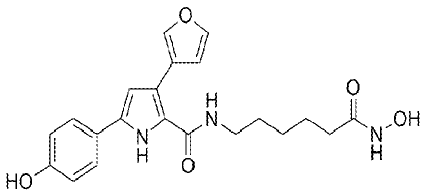
[6] *N*-[5-(Hydroxycarbamoyl)pentyl]-5-(4-hydroxyphenyl)-3-(3-thienyl)-1*H*-pyrrole-2-carboxamide, with the following structural formula: 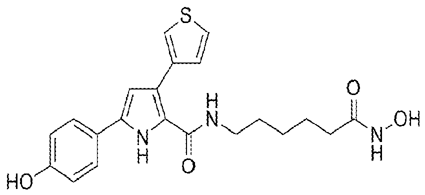 or a salt, solvate or prodrug thereof.

In the context of the present invention, the following terms have the meanings detailed below:
The term "C₆-C₁₀ aryl" refers to an aromatic group having 6 to 10 carbon atoms, comprising 1, 2 or 3 aromatic rings, linked by a carbon-carbon bond or condensed, including for example and in a non limiting sense, phenyl, naphthyl, biphenyl, indenyl, etc. Preferably "aryl" refers to phenyl.
"Heteroaryl" refers to a stable 3- to 10-membered aromatic ring, preferably a 5-or 6-membered aromatic ring, which consists of carbon atoms and from one to five (i.e. one, two, three, four or five) heteroatoms selected from nitrogen, oxygen and sulphur.

For the purposes of this invention, the heteroaryl radical may be a monocyclic, bicyclic or tricyclic ring systems, which may include condensed ring systems; and nitrogen, carbon or sulphur atoms in the heteroaryl radical may be optionally oxidized; and the nitrogen atom may be optionally quaternized. Examples of such heteroaryl include, but are not limited to, benzimidazole, benzothiazole, furan, thiophene, pyrrole, pyridine, pyrimidine, isothiazole, imidazole, indole, purine, quinolone, thiadiazole. Furan and thiophene are preferred heteroaryl groups.

The term "alkyl" refers to a linear or branched hydrocarbon chain radical consisting of carbon and hydrogen atoms, containing no unsaturation, having the carbon atoms indicated in each case, which is attached to the rest of the molecule by a single bond. Preferred alkyl radicals have from 1 to 6 carbon atoms. Exemplary alkyl groups can be methyl, ethyl, n-propyl, or i-propyl.

"Alkoxy" refers to a radical of the formula -O-alkyl where "alkyl" is as defined above, having between 1 and 6 carbon atoms. In an embodiment of the invention alkoxy refers to a radical of formula -O-(C₁-C₃ alkyl). Exemplary alkoxy radicals are methoxy, ethoxy, n-propoxy or i-propoxy.

As commonly understood in this technical area, there may be a degree of substitution in the radicals defined above. Thus, there may be substitution in either group of the present invention. References in this document with respect to substituted groups in the groups of the present invention indicate that the specified radical may be substituted in one or more positions available with one or more substituents. These substituents include, for example and in a non limiting sense, C₁-C₆ alkyl, C₆-C₁₀ aryl, 3-to 10-membered heteroaryl, halogen, cyano, nitro, trifluoromethyl, -N(R')(R"), -OR', - SR', -SOR', -SO₂R', -C(O)R', -C(O)OR', -C(O)N(R')(R"), -OC(O)R'; where R' and R" are independently selected from hydrogen, C₁-C₆ alkyl, C₆-C₁₀ aryl, 3- to 10-membered heteroaryl and trifluoromethyl.

The compounds of formula (I) may be in the form of salts, preferably as pharmaceutically acceptable salts, as solvates or as prodrugs.

The term "pharmaceutically acceptable salts" refers to salts which, when administered to the recipient, can provide (directly or indirectly) a compound as described in the present document. "Pharmaceutically acceptable" preferably refers to compositions and molecular entities that are physiologically tolerable and do not usually produce an allergic reaction or a similar unfavorable reaction as gastric disorders, dizziness and suchlike, when administered to a human or animal. Preferably, the term "pharmaceutically acceptable" means it is approved by a regulatory agency of a state or federal government or is included in the U.S. Pharmacopoeia or other generally recognized pharmacopoeia for use in animals, and more particularly in humans.

The preparation of salts can be accomplished by methods known in the art. For example, the pharmaceutically acceptable salts of compounds provided herein are synthesized from the original compound, which contains basic residues, by conventional chemical methods. Generally, such salts are prepared, for example, by reacting free base forms of these compounds with the appropriate base or acid in water or in an organic solvent or in a mixture of both. In general, non-aqueous media like ether, ethyl acetate, ethanol, isopropanol or acetonitrile are preferred. Examples of acid addition salts include mineral acid addition salts such as, e.g. hydrochloride, hydrobromide, hydroiodide, sulfate, nitrate, phosphate salts and organic acid addition salts such as, for example, acetate, maleate, fumarate, citrate, oxalate, succinate, tartrate, malate, mandelate, methanesulfonate and *p*-toluenesulfonate salts. Examples of base addition salts include inorganic salts such as, for example, sodium, potassium, calcium, ammonium, magnesium, aluminum and lithium salts, and organic salts such as, for example, ethylenediamine, ethanolamine, *N*,*N*-dialkylenethanolamine, triethanolamine, glucamine and basic salts of aminoacids.

The term "prodrug" is used in its broadest sense and encompasses those derivatives that are converted *in vivo* into the compounds of the invention. Experts in the art would readily produce such derivatives, and include, depending on the functional groups present in the molecule and without limitation, the following derivatives of the present compounds: ethers, esters, amino acid esters, phosphate esters, esters of metallic salt sulfonates, carbamates and amides.

The term "solvate" according to this invention is to be understood as any form of the active compound of the invention which has another molecule (for example a polar solvent such as water or ethanol, a cyclodextrin or a dendrimer) attached to it through noncovalent bonds. Examples of solvates include hydrates and alcoholates, for example methanolate. The compounds of the invention can be in crystalline form, either as free compounds or as solvates. Methods of solvation are known within the art. In a particular embodiment the solvate is a hydrate.

Salts, solvates and prodrugs can be prepared by methods known in the state of the art. Note that the non-pharmaceutically acceptable salts, solvates and prodrugs also fall within the scope of the invention because they can be useful in preparing pharmaceutically acceptable salts, solvates or prodrugs.

The compounds of the invention also seek to include compounds that differ only in the presence of one or more isotopically enriched atoms. For example, compounds having the present structures except for the replacement of a hydrogen by a deuterium or tritium, or the replacement of a carbon by a carbon enriched in ¹¹C, ¹³C or ¹⁴C or a ¹⁵N enriched nitrogen are within the scope of this invention.

As noted above, at least one of R¹ and R² in formula (I) is a phenol group (i.e. a hydroxyphenyl group) such as a 2-phenol (i.e. ortho-phenol or 2-hydroxyphenyl), 3-phenol (i.e. meta-phenol or 3-hydroxyphenyl) or 4-phenol group (i.e. para-phenol or 4-hydroxyphenyl). Such groups are independently selected for R¹ and R². Specially preferred embodiments are those wherein R¹ and/or R² represent a 4-phenol group.

In a preferred embodiment of the invention, one of the R¹ and R² in formula (I) represents a phenol group (preferably a 4-phenol group), and the other one represents an optionally substituted phenyl group or an optionally substituted 5- or 6-membered heteroaryl group.

In more preferred embodiment, one of the R¹ and R² in formula (I) represents a phenol group (preferably a 4-phenol group), and the other one represents an optionally substituted phenyl group, an optionally substituted furan or an optionally substituted thiophene. Even more preferably, one of the R¹ and R² in formula (I) represents a phenol group (preferably a 4-phenol group), and the other one represents an optionally substituted phenyl group, an optionally substituted 3-furyl group or an optionally substituted 3-thienyl group.

Another aspect of the invention refers to different procedures to obtain compounds of general formula (I). The following methods A and B describe different procedures for obtaining compounds of general formula (I), or salts, solvates or prodrugs thereof.

### Method A

Method A represents a procedure for the preparation of compounds of general formula (I): 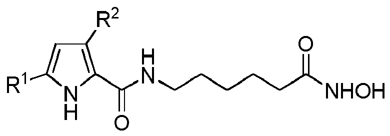 where R¹ and R² have the meaning given above, which comprises reacting:
a) a hydroxyphenyl-substituted 1 H-pyrrole-2-carboxylic acid of formula (II); 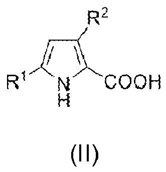
b) a compound of formula (III) or a salt thereof,

   H₂N-(CH₂)₅-R³ (III)

   where R³ is an alkoxycarbonyl;
c) at least one reagent for the activation of the carboxyl group; and
d) at least one tertiary amine, preferably selected from the group consisting of:
   - cyclic or acyclic tertiary amines with aliphatic substituents having from 3 to 10 carbon atoms in total, such as triethylamine, N-methylpyrrolidine or N-methylmorpholine, and
   - tertiary amines having at least an aromatic substituent and from 8 to 15 carbon atoms in total, such as 4-dimethylaminopyridine;
   and adding the resulting product over a mixture of hydroxylamine hydrochloride and phenolphthalein in the presence of an excess of sodium methoxide in methanol.

For the aims of the invention, the reaction mixture made up of the four compounds of phases a) to d) can be made by adding one of the components to the mixture before the other three in an organic solvent and at a temperature of -85°C to +25°C, preferably temperatures near 0°C. Next it is left for a time to complete the reaction, while reaching ambient temperature. Upon completion of the coupling reaction, the ester obtained is added over a mixture of hydroxylamine hydrochloride and phenolphthalein in the presence of an excess of sodium methoxide in methanol. Upon completion of the reaction, after the corresponding treatment, the compounds of general formula (I) are obtained.

The preparation of compounds of formula (II) described above may be performed by a process which comprises reacting a mixture formed by:
a) a α,β-unsaturated carbonylic compound with the following formula (IV): 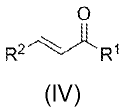 where R¹ and R² have the meanings indicated for compounds of formula (I);
b) an ester of the nitroacetic acid of general formula (V): 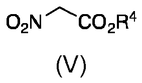 where R⁴ is a C₁-C₆ alkyl group; and
c) a primary, secondary or tertiary amine, or an inorganic base; preferably a cyclic or acyclic aliphatic or monounsaturated tertiary amine. More preferably, triethylamine, *N*,*N*-diisopropyl ethylamine (DIPEA), *N*-methyl morpholine, 1,8-diazabicyclo[5.4.0]undec-7-ene (DBU), or mixtures thereof.

The resulting mixture is preferably stirred at a temperature ranging from about +50°C to +100°C. Upon completion of the reaction, the resulting product, consisting of a mixture of diastereomers of α-nitro-δ-oxoester of general formula (VI): 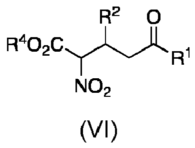 may be oxidized to a compound of general formula (VII). In a particular embodiment the oxidation step is performed in the presence of an oxidant selected from potassium permanganate, dimethyldioxirane, potassium peroxomonosulfate (Oxone^{™}), titanium (III) chloride, m-chloroperoxybenzoic acid (MCPBA), and freshly prepared chromium (II) chloride or mixtures thereof. In a preferred embodiment the compound of general formula (VI) is treated with an excess of sodium methoxide in methanol or sodium ethoxide in ethanol at a temperature ranging from about -25°C to +50°C, preferably at temperatures close to +25°C. The nitronate thus obtained is hydrolyzed for instance in the presence of a strong acid and a protic solvent at a temperature ranging from about -85°C to 0°C, preferably at temperatures close to -25°C. For the purposes of the invention, a preferred embodiment refers to the use of methanol and sulfuric acid to yield the corresponding α,δ-dioxoesters of general formula (VII):

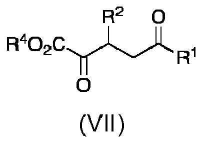

1*H*-Pyrrole-2-carboxylic acids of general formula (II) may be obtained by treatment of previously described esters of formula (VII) with ammonium hydroxide or an ammonium salt of an aliphatic carboxylic acid of less than 5 carbon atoms (such as ammonium acetate, ammonium propionate or ammonium butanoate) in the presence of acetic acid at a preferred temperature ranging from about +25°C to +100°C, and subsequent hydrolysis, preferably in the presence of an alkaline hydroxide, water, an alcohol and a cyclic or acyclic ether, at a temperature between about 0°C and 100°C, preferably at temperatures close to the range from +50°C to +100°C yielding, after the corresponding treatment, the compounds of general formula (II).

### Method B

Method B represents another procedure for the preparation of compounds of general formula (I): 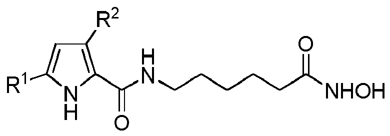 where R¹ and R² have the meaning given above, which comprises reacting:
a) a protected hydroxyphenyl-substituted 1H-pyrrole-2-carboxylic acid of formula (IIP), 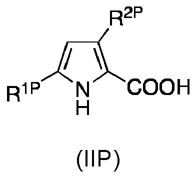 where one of the R^{1P} and R^{2P} represents a protected hydroxyphenyl radical, and the other one represents an optionally substituted C₆-C₁₀ aryl or an optionally substituted 3- to 10-membered heteroaryl group;
b) a compound of formula (III) or a salt thereof,

   H₂N-(CH₂)₅-R³ (III)

   where R³ has the meaning given above;
c) at least one reagent for the activation of the carboxyl group; and
d) at least one tertiary amine, preferably selected from the group consisting of:
   - cyclic or acyclic tertiary amines with aliphatic substituents having from 3 to 10 carbon atoms in total, such as triethylamine, N-methylpyrrolidine or N-methylmorpholine, and
   - tertiary amines having at least an aromatic substituent and from 8 to 15 carbon atoms in total, such as 4-dimethylaminopyridine;
   performing a deprotection reaction to remove the protecting group of the hydroxyphenyl radical, preferably by acid catalysis or by hydrogenation, and adding the resulting product over a mixture of hydroxylamine hydrochloride and phenolphthalein in the presence of an excess of sodium methoxide in methanol.

For the aims of the invention, the reaction mixture made up of the four compounds of phases a) to d) can be made by adding one of the components to the mixture before the other three in an organic solvent and at a temperature of about - 85°C to +25°C, preferably temperatures near 0°C. Next it is left for a time to complete the reaction, while reaching ambient temperature. Once the coupling reaction is completed, the protecting group of the hydroxyphenyl radical is removed preferably by acid catalysis or by hydrogenation. The final step consists of adding the obtained deprotected product over a mixture of hydroxylamine hydrochloride and phenolphthalein in the presence of an excess of sodium methoxide in methanol. Once the reaction is completed, after the corresponding treatment, the compounds of general formula (I) are obtained.

According to a particular embodiment, when using a methoxy group as a hydroxy protecting group in any of the compounds of the invention, the deprotection reaction is carried out preferably by reaction with an acid, preferably a Lewis acid, more preferably boron tribromide, and a suitable organic solvent, preferably a halogenated solvent, more preferably dichloromethane, under dry atmosphere at a temperature ranging from about 0°C to +40°C, preferably at temperatures around 0°C. According to another particular embodiment, when using a benzyl group as a hydroxy protecting group, the deprotection reaction is carried out preferably by hydrogenation.

The preparation of compounds of formula (IIP) described above may be carried out i) in an organic solvent or ii) in its absence and microwave irradiation, and performed by a process which comprises reacting a mixture formed by:
a) a nitroalkene of configuration *E* or *Z* with the following formula (VIII),

   O₂N-CH=CH-R^{2P} (VIII)

   where R^{2P} represents a protected hydroxyphenyl radical, an optionally substituted C₆-C₁₀ aryl or an optionally substituted 3-to 10-membered heteroaryl group;
b) an imine of configuration *E* or *Z* with the following formula (IX),

   R^{1P}-CH=N-CH₂-COOR⁵ (IX)

   where R^{1P} represents a protected hydroxyphenyl radical, an optionally substituted C₆-C₁₀ aryl or an optionally substituted 3-to 10-membered heteroaryl group and R⁵ represents a C₁-C₆ alkyl or C₆-C₁₀ aryl group;
c) a metal salt, preferably selected from lithium perchlorate, silver perchlorate and silver acetate; and
d) at least one tertiary amine, preferably selected from the group consisting of:
   - cyclic or acyclic tertiary amines with aliphatic substituents having from 3 to 10 carbon atoms in total, such as triethylamine, N-methylpyrrolidine or N-methylmorpholine, and
   - tertiary amines having at least an aromatic substituent and from 8 to 15 carbon atoms in total, such as 4-dimethylaminopyridine;
   treating the obtained 2-alkoxycarbonyl pyrrolidine isomers with an oxidizing agent and thermal heating or microwave irradiation, and subjecting the obtained 2-alkoxycarbonyl 1*H*-pyrrole to alkaline hydrolysis.

For the aims of the invention, the reaction mixture made up of the four components indicated above can be carried out using microwave irradiation or by adding one of the components over the other three, in an organic solvent and at a temperature between about -25°C to +25°C, preferably at temperatures near +25°C. The mixture of 2-alkoxycarbonyl pyrrolidine isomers obtained may be solved in a cyclic ether such as tetrahydrofuran or a high boiling point acyclic ether such as bis(2-methoxyethyl) ether, also known as diglyme, and an oxidizing agent is added such as manganese dioxide, hydrogen peroxide or 2,3-dichloro-5,6-dicyano-1,4-benzoquinone. The reaction mixture is stirred at temperatures between about +60°C and +250°C. Alternatively the treatment with an oxidizing agent may be performed under microwave irradiation. Upon completion of the reaction, a mixture made up of 2-alkoxycarbonyl-*NH*-pyrrole and the corresponding 2-alkoxycarbonyl-4-nitro-*NH-*pyrrole is obtained as product, whose components can be separated by fractionated crystallization or chromatography. The acids of formula (IIP) are obtained by means of hydrolysis of the previous purified esters, preferably in the presence of an alkaline hydroxide, water, an alcohol and a cyclic or acyclic ether, at a temperature between about 0°C and 100°C, preferably at temperatures close to the range from +50°C to +100°C yielding, after the corresponding treatment, the compounds of general formula (IIP).

Methods A and B may include protection and deprotection steps of functional groups, if necessary. Protecting groups and methods of protection and deprotection are well known to the skilled in the art. Illustrative examples of such protecting groups are described in Green T.W. et al. "Protective Groups in Organic Synthesis", 3rd Edition (1999), Ed. John Wiley & Sons.

As a common element for methods A and B, the reagent or group of reagents for the activation of the carboxyl group is preferably oxalyl chloride, phenyl dichlorophosphate, diethyl cyanophosphonate (DEPC), or the 1-hydroxybenzotriazole (HOBt) and *N*-(3-dimethylaminopropyl)-*N*'-ethylcarbodiimide (EDC) system. Also, the organic solvent is preferably a polar organic solvent, most preferred is *N*,*N-*dimethylformamide or 1,2-dimethoxyethane, and the tertiary amine is preferably selected among triethylamine, pyridine, *N*-methylpyrrolidine or *N*-methylmorpholine.

Microwaves may be generated using known equipment such as CEM Discover microwave reactor or a Biotage Initiator microwave reactor. More are available in the market and the skilled person may choose the most appropriate. In an embodiment of the invention the reaction takes place by irradiating the mixture with microwaves at a power comprised between about 50W and 1200W, preferably between 100W and 400W, preferably at about 100W. The pressure is typically comprised between about 10 and 500 PSI, preferably between 30 and 200, more preferably between 60 and 100 PSI.

In additional preferred embodiments, the preferences described above for the different substituents in the compounds of the invention as well as for the conditions of the processes for their preparation are combined. The present invention is also directed to such combinations of preferred substitutions in the formula (I) above and conditions of the processes for obtaining the same.

Another aspect of the present invention relates to a compound of general formula (I) or a pharmaceutically acceptable salt, solvate or prodrug thereof, for use as a medicament.

Another aspect of the present invention relates to a compound of general formula (I) or a pharmaceutically acceptable salt, solvate or prodrug thereof, for use as a medicament for the treatment and/or prevention of diseases or disorders responsive or sensitive to the inhibition of histone deacetylases. In a preferred embodiment, the compound of general formula (I) provides a high selectivity in the inhibition of different HDAC enzymes, especially against HDAC6.

Another aspect of the present invention relates to the use of a compound of general formula (I) or a pharmaceutically acceptable salt, solvate or prodrug thereof, in the preparation of a medication for the treatment and/or prevention of diseases or disorders mediated by histone deacetylase.

The mechanism of action of these compounds is explained by their antagonist properties against histone deacetylases involved in the regulation of processes related to apoptosis, cell growth, tumor progression, cancer metastasis, cell adhesion, angiogenesis, microtubule stability and function, molecular chaperon activity, degradation of misfolded proteins and others. These properties prevent the binding of HDACs to their natural ligands, which can be histones or cytoplasmic proteins such as alpha-tubulin, Hsp90, cortactin, the redox regulatory proteins peroxiredoxin (Prx) I and Prx II and p53 among others, as well as their normal catalytic activation, namely the deacetylation of ε-N-acetyl lysine residues present in these proteins.

Diseases or disorders responsive or sensitive to the inhibition of histone deacetylases include, but are not limited to, proliferative diseases, such as cancer, benign prostatic hyperplasia, endometriosis, oral leukoplakia; hematological malignancies; autoimmune diseases; inflammatory diseases (skin inflammatory diseases such as psoriasis, acne or eczema, musculoskeletal inflammatory diseases such as rheumatoid arthritis, juvenile rheumatoid arthritis, ankylosing spondylitis or osteoarthritis, inflammatory conditions of the gastrointestinal tract such as inflammatory bowel disease, Crohn's disease, ulcerative colitis, irritable bowel syndrome); cardiovascular diseases, such as stroke, myocardial infarction, cardiac hypertrophy, chronic heart failure; diseases of the central nervous system (CNS), such as neurodegenerative diseases (*e.g*. Huntington's disease, Rubinstein-Taybi syndrome, Rett syndrome); psychiatric disorders, such as schizophrenia and anxiety; endocrine and metabolic disorders, such as diabetes.

Another aspect of the invention relates to a pharmaceutical composition comprising at least one compound of general formula (I) or a pharmaceutically acceptable salt, solvate or prodrug thereof, and at least one pharmaceutically acceptable excipient.

The compounds of the present invention can be used with at least another drug to provide a combination therapy. This other drug or drugs may be part of the same composition, or may be provided as a separate composition and can be administered at the same time or at different times.

The term "treatment" or "treating" in the context of this document means administration of a compound or a formulation according to this invention to prevent, improve or eliminate the disease or one or more symptoms associated with the disease. "Treatment" also encompasses preventing, improving or eliminating the physiological sequelae of the disease.

The term "excipient" refers to a vehicle, diluent or adjuvant that is administered with the active ingredient. Such pharmaceutical excipients can be sterile liquids, such as water and oils, including those of petroleum, animal, vegetable or synthetic origin, such as peanut oil, soybean oil, mineral oil, sesame oil and similars. Water or saline aqueous solutions and aqueous dextrose and glycerol, particularly for injectable solutions, are preferably used as vehicles. Suitable pharmaceutical vehicles are described in "Remington's Pharmaceutical Sciences" by E.W. Martin, 21st Edition, 1995; or "Handbook of Pharmaceutical Excipients", Rowe C. R.; Paul J. S.; Marian E. Q., sixth Edition.

Examples of pharmaceutical compositions include any solid composition (tablets, pills, capsules, granules, etc.) or liquid composition (solutions, suspensions or emulsions) for oral, topical or parenteral administration.

In a preferred embodiment the pharmaceutical compositions are in oral delivery form. Pharmaceutical forms suitable for oral administration may be tablets and capsules and may contain conventional excipients known in the art such as binders, for example syrup, gum arabic, gelatin, sorbitol, tragacanth or polyvinylpyrrolidone; fillers, for example lactose, sugar, cornstarch, calcium phosphate, sorbitol or glycine; lubricants for the preparation of tablets, for example magnesium stearate; disintegrants, for example starch, polyvinylpyrrolidone, sodium starch glycolate or microcrystalline cellulose; or pharmaceutically acceptable wetting agents such as sodium lauryl sulphate.

Solid oral compositions can be prepared by conventional methods of blending, filling or preparation of tablets. Repeated blending operations can be used to distribute the active ingredient in all the compositions that use large amounts of fillers. Such operations are conventional in the art. The tablets can be prepared, for example, by dry or wet granulation and optionally can be coated by well known methods in normal pharmaceutical practice, in particular using a enteric coating.

Pharmaceutical compositions can also be adapted for parenteral administration, such as sterile solutions, suspensions or lyophilized products in the appropriate unit dosage form. Suitable excipients such as fillers, buffering agents or surfactants can be used.

The mentioned formulations will be prepared using standard methods such as those described or referred to in the Spanish and U.S. Pharmacopoeias and similar reference texts.

In general, the effective amount of a compound of the invention to be administered will depend on the relative efficacy of the compound chosen, the severity of the disorder being treated and the patient's weight. However, the active compounds will normally be administered one or more times a day for example 1, 2, 3 or 4 times daily, with typical total daily doses in the range from 0.01 up to 1000 mg/kg/day.

In order to facilitate the understanding of the preceding ideas, some examples of embodiment of the present invention are described below. These examples are merely illustrative.

### Examples

### Example 1: Preparation of N-(6-(hydroxyamino)-6-oxohexyl)-3-(4-hydroxyphenyl)-5-phenyl-1H-pyrrole-2-carboxamide, with the following structural formula:

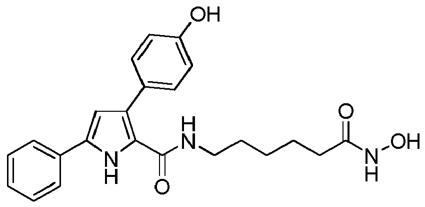

This material was prepared using one of the methods described herein, yielding the title compound.

### Method A:

### 3-(4-Hydroxyphenyl)-5-phenyl-1H-pyrrole-2-carboxylic acid

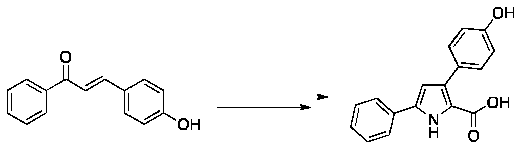

A solution of (2*E*)-3-(4-hydroxyphenyl)-1-phenylprop-2-en-1-one (4.7 g, 20.98 mmol) and ethyl nitroacetate (2.32 ml, 20.98 mmol) in triethylamine (8.77 ml, 62.94 mmol) was stirred at 75°C for 4h. Ethyl acetate (500 ml) was added and the solution obtained was washed with HCl 1N (4 x 250 ml), dried over Na₂SO₄ and evaporated under reduced pressure, to obtain 6.94 g of ethyl 3-(4-hydroxyphenyl)-2-nitro-5-oxo-5-phenyl-pentanoate.

To this material a 0.5 M solution of sodium methoxide in methanol (58.2 ml) was added and the mixture was stirred for 4h. Then it was poured over a mixture of H₂SO₄ (12 ml) and MeOH (59 ml) at -20°C. The resulting mixture was stirred at -20°C for 5 minutes, and then allowed to reach room temperature. H₂O (50 ml) was added, methanol was removed under reduced pressure and the resulting aqueous solution was extracted with CH₂Cl₂ (2 x 1000 ml). The combined organic layers were washed with NaOH (2 x 360 ml, 1% aqueous solution) and with NaCl (2 x 360 ml, saturated aqueous solution), dried over Na₂SO₄ and evaporated under reduced pressure, to obtain 4.92 g of ethyl 3-(4-hydroxyphenyl)-2,5-dioxo-5-phenylpentanoate.

To this material ammonium acetate (4.53 g, 11.75 mmol) and glacial acetic acid (7.2 ml) were added, and the mixture was stirred at 75°C for 1 h. After reaching room temperature, ethyl acetate (800 ml) was added and the resulting solution was washed with NaHCO₃ (3 x 200 ml, saturated aqueous solution), dried over Na₂SO₄ and evaporated under reduced pressure, to obtain 3.13 g of ethyl 3-(4-hydroxyphenyl)- 5-phenyl-1*H*-pyrrole-2-carboxylate. This material was dissolved in ethanol (105 ml) and ethyleneglycol dimethyl ether (3 ml) and 10% NaOH (38.4 ml, aqueous solution) was added dropwise. The resulting mixture was stirred under reflux and the progress of the reaction was monitored by TLC. Upon completion of the reaction, ethanol was removed under reduced pressure and the resulting aqueous solution was cooled to 0°C, neutralized with HCl 6N and extracted with ethyl acetate (3x100 ml). The combined organic layers were dried over Na₂SO₄ and evaporated under reduced pressure, to obtain 3-(4-hydroxyphenyl)-5-phenyl-1*H*-pyrrole-2-carboxylic acid: Yield 53%; m.p. 192-193°C; IR 3466, 3321, 1644, 1504, 1252, 1142, 804, 747 cm⁻¹; ¹H-NMR (500 MHz, δ ppm, DMSO-d₆) 12.14 (s_{b}, 1H), 11.63 (s, 1H), 9.29 (s, 1H), 7.87 (d, *J* = 7.5 Hz, 2H), 7.38 (m, 4H), 7.27 (t, *J* = 7.3 Hz, 1H), 6.74 (d, *J* = 8.3 Hz, 2H), 6.64 (s, 1H); ¹³C-NMR (75 MHz, δ ppm, DMSO-d₆) 162.0, 156.2, 134.9, 132.4, 131.2, 130.4, 128.5, 127.1, 126.1, 125.2, 118.7, 114.4, 109.4; Anal. Calc. for C₁₇H₁₃NO₃: C, 73.11; H, 4.69; N, 5.02. Found: C, 73.29; H, 4.67; N, 4.96%.

### Methyl 6-[3-(4-hydroxyphenyl)-5-phenyl-1H-pyrrole-2-carboxamido] hexanoate

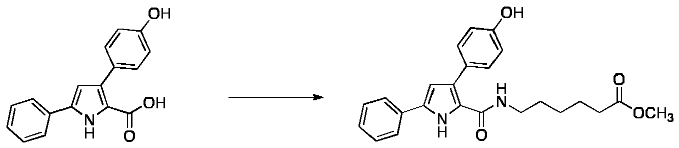

A solution of 3-(4-hydroxyphenyl)-5-phenyl-1H-pyrrole-2-carboxylic acid (1.8 g, 6.5 mmol) and methyl 6-aminohexanoate hydrochloride (1.90 g, 10.5 mmol) in DMF (50 ml) was cooled to 0°C. Triethylamine (7.7 ml, 55.6 mol), 1-hydroxybenzotriazole (1.64 g, 10.7 mmol), *N*-(3-dimethylaminopropyl)-*N*'-ethylcarbodiimide hydrochloride (2.05 g, 10.7 mmol) and *N*-methylmorpholine (1.00 ml, 9.8 mmol) were added subsequently. The mixture was stirred for 2h at 0°C, and for an additional 96h at room temperature. Then ethyl acetate (300 ml) was added, and the obtained solution was washed with water (50 ml), Na₂S₂O₃ 1N (50 ml, aqueous solution), water (50 ml), NaHCO₃ (50 ml, saturated aqueous solution), and NaCl (50 ml, saturated aqueous solution), dried over Na₂SO₄ and evaporated under reduced pressure, to obtain methyl 6-[3-(4-hydroxyphenyl)-5-phenyl-1H-pyrrole-2-carboxamido] hexanoate: Yield 43%; m.p. 142-143°C; IR (KBr) 3413, 3355, 2932, 1739, 1623, 1168 cm⁻¹; ¹H-NMR (500 MHz, δ ppm, DMSO-d₆) 8.54 (s, 1H), 7.82 (d, *J* = 7.4 Hz, 2H), 7.51 (s, 1H), 7.36 (t, *J* = 7.7 Hz, 2H), 7.29 (d, *J* = 8.4 Hz, 2H), 7.21 (t, *J* = 7.3 Hz, 1H), 6.75 (d, *J* = 8.5 Hz, 2H), 6.56 (s, 1H), 3.58 (s, 3H), 3.19 - 3.10 (m, 3H), 2.28 (t, *J* = 7.4 Hz, 2H), 1.52 (dt, *J* = 15.0 Hz, *J*' = 7.4 Hz, 2H), 1.41 (dd, *J* = 14.4 Hz, *J'* = 7.1 Hz, 2H), 1.28 - 1.19 (m, 2H); ¹³C-NMR (126 MHz, δ ppm, DMSO-d₆) 174.5, 173.2, 165.9, 160.9, 156.6, 132.6, 131.8, 129.9, 128.5, 128.0, 126.4, 125.9, 124.5, 122.9, 114.8, 108.2, 51.0, 38.3, 33.1, 28.7, 25.9, 24.1. Anal. Calcd. For C₂₄H₂₆N₂O₄: C, 70.9; H, 6.5; N, 6.9. Found: C, 70.7; H, 6.5; N, 6.9.

### N-(6-(hydroxyamino)-6-oxohexyl)-3-(4-hydroxyphenyl)-5-phenyl-1H-pyrrole-2-carboxamide

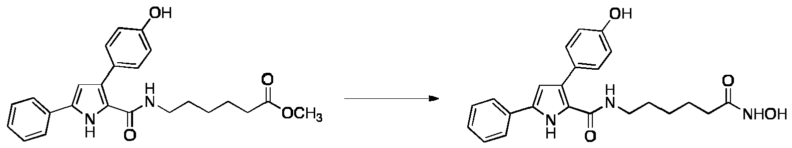

To a solution of hydroxylamine hydrochloride (0.36 g, 5.6 mmol) and phenolphtalein (1 mg) in methanol (1 ml) under inert atmosphere, an aliquot of sodium methoxide in methanol (taken from a solution of 2.70 g, 50 mmol of sodium methoxide in 10 ml of methanol) was added dropwise until a permanent pink color was observed. Methyl 6-[3-(4-hydroxyphenyl)-5-phenyl-1H-pyrrole-2-carboxamido] hexanoate (1 mmol) and sodium methoxide in methanol (7.5 mmol, 0.75 ml of the previously prepared solution) were subsequently added. The reaction mixture was stirred for 26h, the formation of a dense precipitate being observed. Water (3 ml) was added, and this solution was acidified with glacial acetic acid and extracted with CH₂Cl₂ (3 x 10 ml). The combined organic fractions were dried over Na₂SO₄ and evaporated under reduced pressure, to obtain N-(6-(hydroxyamino)-6-oxohexyl)-3-(4-hydroxyphenyl)-5-phenyl-1H-pyrrole-2-carboxamide: Yield 89%; m.p. 99-100°C; IR (KBr) 3401, 3228, 1615, 1542 cm⁻¹; ¹H-NMR (500 MHz, δ ppm, DMSO-d₆) 11.46 (s, 1H), 10.32 (s, 1H), 9.38 (s, 1H), 8.65 (s, 1H), 7.79 (d, *J* = 7.8 Hz, 2H), 7.38 (t, *J* = 7.6 Hz, 2H), 7.30 (d, *J* = 8.4 Hz, 2H), 7.23 (t, *J* = 7.3 Hz, 1H), 7.08 (t, *J* = 5.3 Hz, 1H), 6.77 (d, *J* = 8.4 Hz, 2H), 6.59 (d, *J* = 2.5 Hz, 1H), 3.15 (dd, *J* = 12.7 Hz, *J'* = 6.6 Hz, 2H), 1.93 (dd, *J* = 14.4 Hz, *J'* = 7.1 Hz, 2H), 1.48 (dt, *J* = 15.0 Hz, *J'* = 7.5 Hz, 2H), 1.40 (dt, *J* = 14.4 Hz, *J'* = 7.0 Hz, 2H), 1.25 - 1.16 (m, 2H); ¹³C-NMR (126 MHz, δ ppm, DMSO-d₆) 168.9, 160.9, 156.2, 132.6, 131.6, 130.3, 129.9, 128.5, 127.5, 126.5, 126.1, 125.1, 124.5, 122.9, 114.9, 114.3, 108.2, 38.5, 32.1, 28.7, 26.0, 24.8. Anal. Calcd. For C₂₃H₂₅N₃O₄: C, 67.8; H, 6.2; N, 10.3. Found: C, 67.9; H, 6.1; N, 10.1.

### Method B:

### B.1 Using methyl ether as protecting group, and microwave irradiation for pyrrole obtention

### 3-(4-Methoxyphenyl)-5-phenyl-1H-pyrrole-2-carboxylic acid

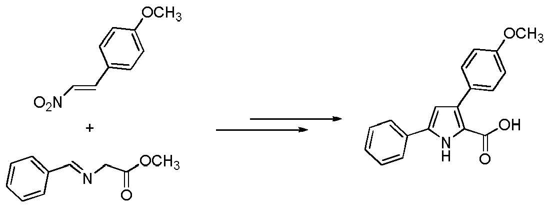

To a mixture of (*E*)-methyl 2-(benzylideneamino)acetate (10.27 g, 58.0 mmol) in acetonitrile (590 ml), triethylamine (8.2 ml, 58.0 mmol), silver acetate (0.47 g, 2.8 mmol) and 1-methoxy-4-((*E*)-2-nitrovinyl)benzene (10.39 g, 58.0 mmol) were added. The reaction mixture was stirred at room temperature, and the progress of the reaction was monitored by TLC. After completion of the reaction, the mixture was filtered through a Celite pad and washed with NH₄Cl saturated water solution (2 x 150 ml) and water (2 x 150 ml). The organic layer was dried over MgSO₄ and evaporated under reduced pressure, yielding a diastereomeric mixture of the corresponding cycloadducts *exo* and *endo*. The mixture of diastereoisomers (10 mmol), 2,3-dichloro-5,6-dicyano-*p-*benzoquinone (DDQ, 15 mmol) and xylene (30 ml) were irradiated in a monomode microwave reactor at 100°C and 255 W for 45 min. The resulting mixture was filtered through a Celite pad. The filtrate was concentrated under reduced pressure to yield a 1:3 mixture of 5-phenyl-3-(4-methoxyphenyl)-2-methoxycarbonyl-4-nitro-1*H*-pyrrole and 5-phenyl-3-(4-methoxyphenyl)-2-methoxycarbonyl-1*H*-pyrrole. The products were separated by flash column chromatography. Purified 5-phenyl-3-(4-methoxyphenyl)-2-methoxycarbonyl-1*H*-pyrrole was dissolved in ethanol (105 ml) and ethyleneglycol dimethyl ether (3 ml) and 10% NaOH (38.4 ml, aqueous solution) was added dropwise. The resulting mixture was stirred under reflux and the progress of the reaction was monitored by TLC. Upon completion of the reaction, ethanol was removed under reduced pressure and the resulting aqueous solution was cooled to 0°C, neutralized with HCl 6N and extracted with ethyl acetate (3 x 100 ml). The combined organic layers were dried over Na₂SO₄ and evaporated under reduced pressure, to obtain 3-(4-methoxyphenyl)-5-phenyl-1H-pyrrole-2-carboxylic acid: Yield 41%; m.p. 198°C (dec.); IR 3467, 1643 cm⁻¹; ¹H-NMR (500 MHz, δ ppm, DMSO-d₆) 11.72 (s, 1H), 7.88 (d, 2H, *J* = 7.7 Hz), 7.51 (d, 2H, *J* = 8.4 Hz), 7.38 (t, 2H, *J* = 7.6 Hz), 7.26 (t, 1H, *J* = 7.2 Hz), 6.91 (d, 2H, *J* = 8.4 Hz), 6.69 (s, 1H), 3.78 (s, 3H), 3.34 (s_{b}, 1H); ¹³C-NMR (126 MHz, δ ppm, DMSO-d₆) 162.5, 157.9, 134.4, 131.3, 131.2, 130.3, 128.6, 127.9, 127.0, 125.1, 119.8, 113.0, 112.9, 109.3, 55.0, 54.9; Anal. Calc. for C₁₈H₁₅NO₃: C, 73.71; H, 5.15; N, 4.78. Found: C, 73.56; H, 5.08; N, 4.81%.

### Methyl 6-[3-(4-methoxyphenyl)-5-phenyl-1H-pyrrole-2-carboxamido] hexanoate

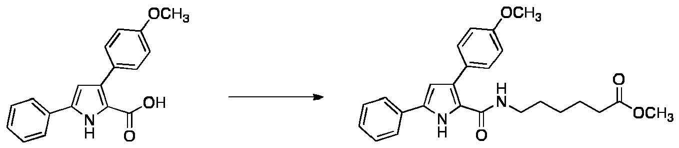

A solution of 3-(4-methoxyphenyl)-5-phenyl-1H-pyrrole-2-carboxylic acid (1.91 g, 6.5 mmol) and methyl 6-aminohexanoate hydrochloride (1.90 g, 10.5 mmol) in DMF (50 ml) was cooled to 0°C. Triethylamine (7.7 ml, 55.6 mol), 1-hydroxybenzotriazole (1.64 g, 10.7 mmol), *N*-(3-dimethylaminopropyl)-*N*'-ethylcarbodiimide hydrochloride (2.05 g, 10.7 mmol) and *N*-methylmorpholine (1.00 ml, 9.8 mmol) were added subsequently. The mixture was stirred for 2h at 0°C, and for an additional 96h at room temperature. Then ethyl acetate (300 ml) was added, and the obtained solution was washed with water (50 ml), Na₂S₂O₃ 1N (50 ml, aqueous solution), water (50 ml), NaHCO₃ (50 ml, saturated aqueous solution), and NaCl (50 ml, saturated aqueous solution), dried over Na₂SO₄ and evaporated under reduced pressure, to obtain methyl 6-[3-(4-methoxyphenyl)-5-phenyl-1H-pyrrole-2-carboxamido] hexanoate: Yield 47%; m.p. 98-99°C; IR (KBr) 3397, 1738, 1623 cm⁻¹; ¹H-NMR (300 MHz, δ ppm, CDCl₃) 9.63 (s, 1H), 7.58 (d, *J* = 7.6 Hz, 2H), 7.41-7.38 (m, 4H), 7.30-7.25 (m, 1H), 7.00 (d, *J* = 8.4 Hz, 2H), 6.47 (d, *J* = 2.9 Hz, 1H), 5.77 (t, *J* = 4.9 Hz, 1H), 3.87 (s, 3H), 3.66 (s, 3H), 3.25 (dd, *J* = 12.9 Hz, *J'* = 6.6 Hz, 2H), 2.26 (t, *J* = 7.5 Hz, 2H), 1.60 - 1.54 (m, 2H), 1.41 - 1.35 (m, 2H), 1.21 - 1.15 (m, 2H); ¹³C-NMR (126 MHz, δ ppm, CDCl₃) 173.9, 161.3, 159.3, 133.7, 131.4, 130.6, 128.9, 127.7, 127.3, 124.6, 122.5, 114.3, 109.4, 55.3, 51.5, 38.9, 33.8, 29.1, 26.3, 24.5; Anal. Calc. for C₂₅H₂₈N₂O₄: C, 71.41; H, 6.71; N, 6.66. Found: C, 71.47; H, 6.74; N, 6.66%.

### Methyl 6-[3-(4-hydroxyphenyl)-5-phenyl-1H-pyrrole-2-carboxamido] hexanoate

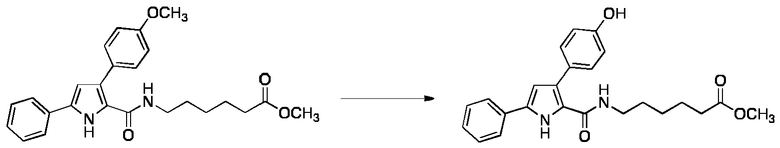

Boron tribromide (1M in dichloromethane, 4 equivalents per methoxy group to be deprotected) was added dropwise to a solution of methyl 6-[3-(4-methoxyphenyl)-5-phenyl-1H-pyrrole-2-carboxamido] hexanoate (0.25 g, 0.6 mmol) in anhydrous dichloromethane (5 ml) at 0°C under argon atmosphere. The mixture was stirred for 16 hours at room temperature and then methanol (approximately 1ml per ml of boron tribromide solution used) was added dropwise at 0°C. The resulting mixture was purified by column cromatography on silicagel using methanol:dichloromethane 1:20 as eluent to obtain methyl 6-[3-(4-hydroxyphenyl)-5-phenyl-1H-pyrrole-2-carboxamido] hexanoate: Yield 46%; m.p. 142-143°C; IR (KBr) 3413, 3355, 2932, 1739, 1623, 1168 cm⁻¹; ¹H-NMR (500 MHz, δ ppm, DMSO-d₆) 8.54 (s, 1H), 7.82 (d, *J* = 7.4 Hz, 2H), 7.51 (s, 1H), 7.36 (t, J = 7.7 Hz, 2H), 7.29 (d, J = 8.4 Hz, 2H), 7.21 (t, J = 7.3 Hz, 1H), 6.75 (d, J = 8.5 Hz, 2H), 6.56 (s, 1H), 3.58 (s, 3H), 3.19 - 3.10 (m, 3H), 2.28 (t, J = 7.4 Hz, 2H), 1.52 (dt, J = 15.0, 7.4, 2H), 1.41 (dd, J = 14.4, 7.1, 2H), 1.28 - 1.19 (m, 2H ).¹³C NMR (126 MHz, δ ppm, DMSO-d₆) 174.5, 173.2, 165.9, 160.9, 156.6, 132.6, 131.8, 129.9, 128.5, 128.0, 126.4, 125.9, 124.5, 122.9, 114.8, 108.2, 51.0, 38.3, 33.1, 28.7, 25.9, 24.1. Anal. Calcd. For C₂₄H₂₆N₂O₄: C, 70.92; H, 6.45; N, 6.89. Found: C, 70.85; H, 6.50; N, 6.91.

### N-(6-(hydroxyamino)-6-oxohexyl)-3-(4-hydroxyphenyl)-5-phenyl-1H-pyrrole-2-carboxamide

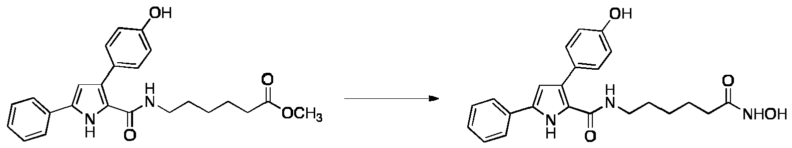

To a solution of hydroxylamine hydrochloride (0.36 g, 5.6 mmol) and phenolphtalein (1 mg) in methanol (1 ml) under inert atmosphere, an aliquot of sodium methoxide in methanol (taken from a solution of 2.70 g, 50 mmol of sodium methoxide in 10 ml of methanol) was added dropwise until a permanent pink color was observed. Methyl 6-[3-(4-hydroxyphenyl)-5-phenyl-1H-pyrrole-2-carboxamido] hexanoate (1 mmol) and sodium methoxide in methanol (7.5 mmol, 0.75 ml of the previously prepared solution) were subsequently added. The reaction mixture was stirred for 26h, the formation of a dense precipitate being observed. Water (3 ml) was added, and this solution was acidified with glacial acetic acid and extracted with CH₂Cl₂ (3 x 10 ml). The combined organic fractions were dried over Na₂SO₄ and evaporated under reduced pressure, to obtain N-(6-(hydroxyamino)-6-oxohexyl)-3-(4-hydroxyphenyl)-5-phenyl-1H-pyrrole-2-carboxamide: Yield 89%; m.p. 99-100°C; IR (KBr) 3401, 3228, 1615, 1542 cm⁻¹; ¹H-NMR (500 MHz, δ ppm, DMSO-d₆) 11.46 (s, 1H), 10.32 (s, 1H), 9.38 (s, 1H), 8.65 (s, 1H), 7.79 (d, *J* = 7.8 Hz, 2H), 7.38 (t, *J* = 7.6 Hz, 2H), 7.30 (d, *J* = 8.4 Hz, 2H), 7.23 (t, *J* = 7.3 Hz, 1H), 7.08 (t, *J* = 5.3 Hz, 1H), 6.77 (d, *J* = 8.4 Hz, 2H), 6.59 (d, *J* = 2.5 Hz, 1H), 3.15 (dd, *J* = 12.7 Hz, *J'* = 6.6 Hz, 2H), 1.93 (dd, *J* = 14.4 Hz, *J'* = 7.1 Hz, 2H), 1.48 (dt, *J* = 15.0 Hz, *J'* = 7.5 Hz, 2H), 1.40 (dt, *J* = 14.4 Hz, *J'* = 7.0 Hz, 2H), 1.25 - 1.16 (m, 2H); ¹³C-NMR (126 MHz, δ ppm, DMSO-d₆) 168.9, 160.9, 156.2, 132.6, 131.6, 130.3, 129.9, 128.5, 127.5, 126.5, 126.1, 125.1, 124.5, 122.9, 114.9, 114.3, 108.2, 38.5, 32.1, 28.7, 26.0, 24.8. Anal. Calcd. For C₂₃H₂₅N₃O₄: C, 67.80; H, 6.18; N, 10.31. Found: C, 67.89; H, 6.16; N, 10.19.

### B.2 Using benzyl ether as protecting group, and thermal oxidation for pyrrole obtention 3-(4-Benzyloxyphenyl)-5-phenyl-1H-pyrrole-2-carboxylic acid

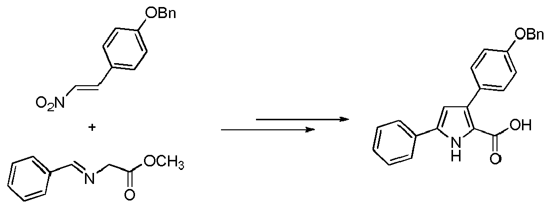

To a mixture of (*E*)-methyl 2-(benzylideneamino)acetate (10.27 g, 58.0 mmol) in acetonitrile (590 ml), triethylamine (8.2 ml, 58.0 mmol), silver acetate (0.47 g, 2.8 mmol) and 1-benzyloxy-4-((*E*)-2-nitrovinyl)benzene (14.80 g, 58.0 mmol) were added. The reaction mixture was stirred at room temperature, and the progress of the reaction was monitored by TLC. After completion of the reaction, the mixture was filtered through a Celite pad and washed with NH₄Cl saturated water solution (2 x 150 ml) and water (2 x 150 ml). The organic layer was dried over MgSO₄ and evaporated under reduced pressure, yielding a diastereomeric mixture of the corresponding cycloadducts *exo* and *endo*. The mixture of diastereoisomers (10 mmol) was solved in diglyme (100 ml) under inert atmosphere and MnO₂ was added (8.9 g, 100 mmol). The reaction mixture was refluxed for 48h. After completion of the reaction, the crude mixture was filtered through a Celite pad, and the filtrate was concentrated under reduced pressure to yield a mixture of 5-phenyl-3-(4-benzyloxyphenyl)-2-methoxycarbonyl-4-nitro-1*H-*pyrrole and 5-phenyl-3-(4-benzyloxyphenyl)-2-methoxycarbonyl-1*H*-pyrrole. The products were separated by flash column chromatography.

Purified 3-(4-benzyloxyphenyl)-5-phenyl-2-methoxycarbonyl-1*H*-pyrrole was dissolved in ethanol (105 ml) and ethyleneglycol dimethyl ether (3 ml) and 10% NaOH (38.4 ml, aqueous solution) was added dropwise. The resulting mixture was stirred under reflux and the progress of the reaction was monitored by TLC. Upon completion of the reaction, ethanol was removed under reduced pressure and the resulting aqueous solution was cooled to 0°C, neutralized with HCl 6N and extracted with ethyl acetate (3 x 100 ml). The combined organic layers were dried over Na₂SO₄ and evaporated under reduced pressure, to obtain 3-(4-benzyloxyphenyl)-5-phenyl-1H-pyrrole-2-carboxylic acid: Yield 37%; m.p. 120-122°C; IR 3371, 1604, 1229, 1024 cm⁻¹; ¹H-NMR (500 MHz, δ ppm, DMSO-d₆) 10.91 (s, 1H), 7.78 (dd, *J* = 22.1 Hz, *J'* = 7.5 Hz, 4H), 7.49 - 7.35 (m, 5H), 7.33 (d, *J* = 7.0 Hz, 1H), 7.26 (s, 2H), 7.14 (d, *J* = 6.6 Hz, 1H), 6.87 (d, *J* = 7.7 Hz, 2H), 6.63 (s, 1H), 5.07 (s, 2H); ¹³C-NMR (126 MHz, δ ppm, DMSO-d₆) 166.1, 156.2, 137.4, 132.3, 130.1, 129.6, 128.5, 128.3, 127.6, 127.5, 125.8, 124.1, 113.5, 107.6. Anal. Calcd. For C₂₄H₁₉NO₃: C, 78.03; H, 5.18; N, 3.79. Found: C, 78.11; H, 5.25; N, 3.82.

### Methyl 6-[3-(4-benzyloxyphenyl)-5-phenyl-1H-pyrrole-2-carboxamido] hexanoate

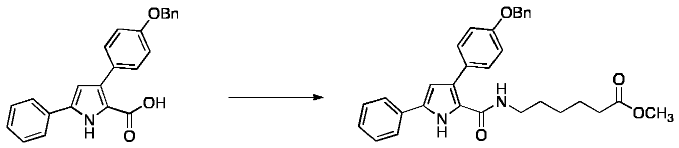

A solution of 3-(4-benzyloxyphenyl)-5-phenyl-1H-pyrrole-2-carboxylic acid (2.4 g, 6.5 mmol) and methyl 6-aminohexanoate hydrochloride (1.90 g, 10.5 mmol) in DMF (50 ml) was cooled to 0°C. Triethylamine (7.7 ml, 55.6 mol), 1-hydroxybenzotriazole (1.64 g, 10.7 mmol), *N*-(3-dimethylaminopropyl)-*N'*-ethylcarbodiimide hydrochloride (2.05 g, 10.7 mmol) and *N*-methylmorpholine (1.00 ml, 9.8 mmol) were added subsequently. The mixture was stirred for 2h at 0°C, and for an additional 96h at room temperature. Then ethyl acetate (300 ml) was added, and the obtained solution was washed with water (50 ml), Na₂S₂O₃ 1 N (50 ml, aqueous solution), water (50 ml), NaHCO₃ (50 ml, saturated aqueous solution), and NaCl (50 ml, saturated aqueous solution), dried over Na₂SO₄ and evaporated under reduced pressure, to obtain methyl 6-[3-(4-benzyloxyphenyl)-5-phenyl-1H-pyrrole-2-carboxamido]hexanoate: Yield 37%; m.p. 130-131°C; IR (KBr) 3321, 3086, 2952, 1661, 1647, 1235, 1000 cm⁻¹; ¹H-NMR (500 MHz, δ ppm, CDCl₃) 9.73 (s, 1H), 7.58 (d, *J* = 7.3 Hz, 2H), 7.47 (d, *J* = 7.3 Hz, 2H), 7.43 - 7.33 (m, 7H), 7.27 (dd, *J* = 12.3 Hz, *J'* = 4.8 Hz, 1H), 7.07 (d, *J* = 8.6 Hz, 2H), 6.47 (d, *J* = 3.0 Hz, 1H), 5.77 (t, *J* = 5.5 Hz, 1H), 5.20 - 5.10 (m, 2H), 3.70 - 3.59 (s, 3H), 3.25 (dd, *J* = 12.8 Hz, *J'* = 6.8 Hz, 2H), 2.27 (t, *J* = 7.5 Hz, 2H), 1.61 - 1.53 (m, 2H), 1.40 - 1.33 (m, 2H), 1.22 - 1.15 (m, 2H); ¹³C-NMR (126 MHz, δ ppm, CDCl₃) 174.1, 161.5, 158.8, 137.0, 134.0, 131.7, 130.9, 129.1, 128.8, 128.4, 128.3, 127.7, 127.5, 127.5, 124.8, 122.8, 115.6, 109.7, 70.4, 51.6, 39.2, 34.1, 29.3, 26.5, 24.7; Anal. Calcd. For C₃₁H₃₂N₂O₄: C, 74.98; H, 6.50; N, 5.64. Found: C, 74.89; H, 6.41; N, 5.77.

### Methyl 6-[3-(4-hydroxyphenyl)-5-phenyl-1H-pyrrole-2-carboxamido] hexanoate

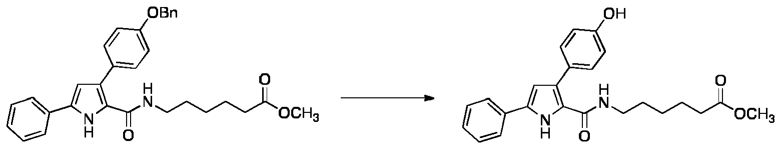

A mixture of methyl 6-[3-(4-benzyloxyphenyl)-5-phenyl-1H-pyrrole-2-carboxamido] hexanoate (0.37 g, 0.74 mmol) and 10% Pd/C (0.08 mg) in dry MeOH (75 mL) was stirred at room temperature under hydrogen atmosphere for 12 h. The palladium catalyst was removed by filtration through a Celite pad and the filtrate was concentrated under reduced pressure to obtain methyl 6-[3-(4-hydroxyphenyl)-5-phenyl-1H-pyrrole-2-carboxamido] hexanoate: Yield 91%; m.p. 142-143°C; IR (KBr) 3413, 3355, 2932, 1739, 1623, 1168 cm⁻¹; ¹H-NMR (500 MHz, δ ppm, DMSO-d₆) 8.54 (s, 1H), 7.82 (d, *J* = 7.4 Hz, 2H), 7.51 (s, 1H), 7.36 (t, *J* = 7.7 Hz, 2H), 7.29 (d, *J* = 8.4 Hz, 2H), 7.21 (t, *J* = 7.3 Hz, 1H), 6.75 (d, *J* = 8.5 Hz, 2H), 6.56 (s, 1H), 3.58 (s, 3H), 3.19 - 3.10 (m, 3H), 2.28 (t, *J* = 7.4 Hz, 2H), 1.52 (dt, *J* = 15.0 Hz, *J'* = 7.4 Hz, 2H), 1.41 (dd, *J* = 14.4 Hz, *J'* = 7.1 Hz, 2H), 1.28 - 1.19 (m, 2H); ¹³C-NMR (126 MHz, δ ppm, DMSO-d₆) 174.5, 173.2, 165.9, 160.9, 156.6, 132.6, 131.8, 129.9, 128.5, 128.0, 126.4, 125.9, 124.5, 122.9, 114.8, 108.2, 51.0, 38.3, 33.1, 28.7, 25.9, 24.1. Anal. Calcd. For C₂₄H₂₆N₂O₄: C, 70.92; H, 6.45; N, 6.89. Found: C, 70.85; H, 6.50; N, 6.91.

### N-(6-(hydroxyamino)-6-oxohexyl)-3-(4-hydroxyphenyl)-5-phenyl-1H-pyrrole-2-carboxamide

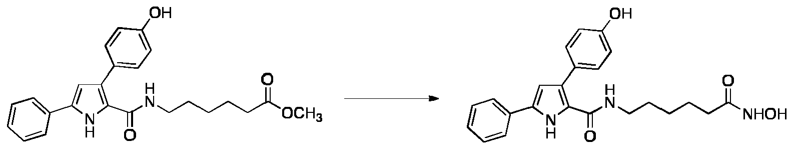

To a solution of hydroxylamine hydrochloride (0.36 g, 5.6 mmol) and phenolphtalein (1 mg) in methanol (1 ml) under inert atmosphere, an aliquot of sodium methoxide in methanol (taken from a solution of 2.70 g, 50 mmol of sodium methoxide in 10 ml of methanol) was added dropwise until a permanent pink color was observed. Methyl 6-[3-(4-hydroxyphenyl)-5-phenyl-1H-pyrrole-2-carboxamido] hexanoate (1 mmol) and sodium methoxide in methanol (7.5 mmol, 0.75 ml of the previously prepared solution) were subsequently added. The reaction mixture was stirred for 26h, the formation of a dense precipitate being observed. Water (3 ml) was added, and this solution was acidified with glacial acetic acid and extracted with CH₂Cl₂ (3 x 10 ml). The combined organic fractions were dried over Na₂SO₄ and evaporated under reduced pressure, to obtain *N*-(6-(hydroxyamino)-6-oxohexyl)-3-(4-hydroxyphenyl)-5-phenyl-1H-pyrrole-2-carboxamide: Yield 89%; m.p. 99-100°C; IR (KBr) 3401, 3228, 1615, 1542 cm⁻¹; ¹H-NMR (500 MHz, δ ppm, DMSO-d₆) 11.46 (s, 1H), 10.32 (s, 1H), 9.38 (s, 1H), 8.65 (s, 1H), 7.79 (d, *J* = 7.8 Hz, 2H), 7.38 (t, *J* = 7.6 Hz, 2H), 7.30 (d, *J* = 8.4 Hz, 2H), 7.23 (t, *J* = 7.3 Hz, 1H), 7.08 (t, *J* = 5.3 Hz, 1H), 6.77 (d, *J* = 8.4 Hz, 2H), 6.59 (d, *J* = 2.5 Hz, 1H), 3.15 (dd, *J* = 12.7 Hz, *J'* = 6.6 Hz, 2H), 1.93 (dd, *J* = 14.4 Hz, *J'* = 7.1 Hz, 2H), 1.48 (dt, *J* = 15.0 Hz, *J'* = 7.5 Hz, 2H), 1.40 (dt, *J* = 14.4 Hz, *J'* = 7.0 Hz, 2H), 1.25 - 1.16 (m, 2H); ¹³C-NMR (126 MHz, δ ppm, DMSO-d₆) 168.9, 160.9, 156.2, 132.6, 131.6, 130.3, 129.9, 128.5, 127.5, 126.5, 126.1, 125.1, 124.5, 122.9, 114.9, 114.3, 108.2, 38.5, 32.1, 28.7, 26.0, 24.8. Anal. Calcd. For C₂₃H₂₅N₃O₄: C, 67.80; H, 6.18; N, 10.31. Found: C, 67.89; H, 6.16; N, 10.19.

### Example 2: Preparation of N-(6-(hydroxyamino)-6-oxohexyl)-5-(4-hydroxyphenyl)-3-phenyl-1H-pyrrole-2-carboxamide, with the following structural formula:

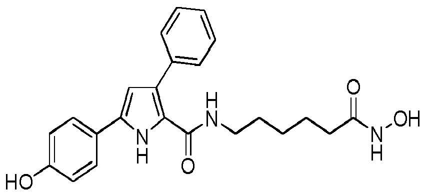

This material was prepared using a method substantially similar to that of Example 1, Method A, yielding the title compound *N*-(6-(hydroxyamino)-6-oxohexyl)-5-(4-hydroxyphenyl)-3-phenyl-1H-pyrrole-2-carboxamide: Yield 85 %; m.p. 224-225°C; IR 3408, 3258, 1679, 1638, 1524, 1286 cm⁻¹; ¹H-NMR (300 MHz, δ ppm, DMSO-d₆) 11.29 (s, 1H), 10.31 (s, 1H), 9.47 (s, 1H), 8.61 (s, 1H), 7.60 (d, *J* = 8.7 Hz, 2H), 7.49 (d, *J* = 7.0 Hz, 2H), 7.35 (t, *J* = 7.4 Hz, 2H), 7.27 (dt, *J* = 4.6 Hz, *J'* = 1.8 Hz, 1H), 7.21 (s, 1H), 6.79 (d, *J* = 8.7 Hz, 2H), 6.47 (d, *J* = 2.7 Hz, 1H), 3.15 (dd, *J* = 12.7 Hz, *J'* = 6.7 Hz, 2H), 1.93 (t, *J* = 7.3 Hz, 2H), 1.53 - 1.36 (m, 4H), 1.27 - 1.20 (m, 2H); ¹³C-NMR (126 MHz, δ ppm, DMSO-d₆) 169.7, 161.6, 157.2, 136.5, 134.0, 129.4, 128.5, 128.3, 126.8, 126.7, 123.4, 122.8, 116.0, 107.4, 39.1, 32.8, 29.3, 26.6, 25.4.

### Example 3: Preparation of N-(5-(Hydroxycarbamoyl)pentyl)-3-(4-hydroxyphenyl)-5-(3-thienyl)-1H-pyrrole-2-carboxamide, with the following structural formula:

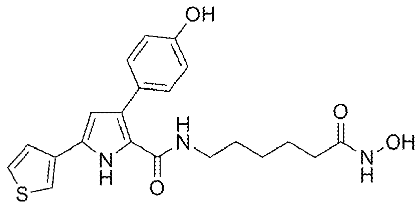

This compound was prepared using a method substantially similar to that of Example 1, Method A: Yield 93%; m.p. 100-102°C; IR 3396, 3238, 1653, 1621, 1547, 1272 cm⁻¹; ¹H-NMR (500 MHz, δ ppm, DMSO-d₆) 11.53 (s, 1H), 7.89 (s, 1H), 7.55 (d, *J* = 1.9 Hz, 2H), 7.27 (d, *J* = 8.5 Hz, 2H), 6.83 (t_{b}, *J* = 4.9 Hz, 1H), 6.79 (d, *J* = 8.5 Hz, 2H), 6.47 (s, 1H), 3.14 (dd, *J* = 12.6 Hz, *J'* = 6.4 Hz, 2H), 1.94 (t, *J* = 7.3 Hz, 2H), 1.50 - 1.44 (m, 2H), 1.41 - 1.34 (m, 2H), 1.21 - 1-15 (m, 2H); ¹³C-NMR (126 MHz, δ ppm, DMSO-d₆) 169.0, 161.0, 156.3, 133.4, 129.9, 129.3, 127.1, 126.3, 126.1, 125.9, 121.9, 118.7, 115.1, 108.3, 38.4, 32.2, 28.7, 26.0, 24.8.

### Example 4: Preparation of 5-(3-Furyl)-N-(5-(Hydroxycarbamoyl)pentyl)-3-(4-hydroxyphenyl)-1H-pyrrole-2-carboxamide, with the following structural formula:

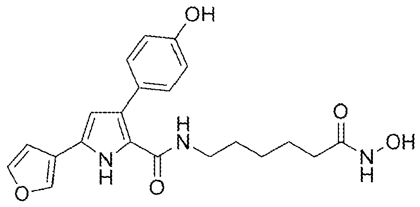

This compound was prepared using a method substantially similar to that of Example 1, Method A: Yield 82%; m.p. 137-139°C; IR 3384, 3220, 1612, 1560, 1296 cm⁻¹; ¹H-NMR (500 MHz, δ ppm, DMSO-d₆) 11.46 (s, 1H), 10.30 (s, 1H), 9.40 (s, 1H), 8.62 (s, 1H), 8.14 (s, 1H), 7.66 (s, 1H), 7.25 (d, *J* = 8.4 Hz, 2H), 6.93 (s, 1H), 6.80 (d, *J* = 8.4 Hz, 2H), 6.60 (t, *J* = 5.2 Hz, 1H), 6.35 (d, *J* = 2.5 Hz, 1H), 3.13 (dd, *J* = 12.6 Hz, *J'* = 6.4 Hz, 2H), 2.05 - 1.91 (m, 2H), 1.55 - 1.41 (m, 2H), 1.41 - 1.30 (m, 2H), 1.22 - 1.13 (m, 2H); ¹³C-NMR (126 MHz, δ ppm, DMSO-d₆) 169.1, 160.9, 156.4, 143.5, 138.5, 129.9, 126.81, 126.1, 125.7, 121.8, 118.3, 115.2, 108.7, 108.2, 48.5, 38.4, 32.2, 29.6, 28.7, 25.9, 24.8, 19.4.

### Example 5: Preparation of 3-(3-Furyl)-N-(5-(hydroxycarbamoyl)pentyl)-5-(4-hydroxyphenyl)-1H-pyrrole-2-carboxamide, with the following structural formula:

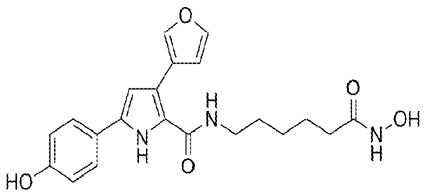

This compound was prepared using a method substantially similar to that of Example 1: Yield 92%; m.p. 73-75°C; IR 3417, 3226, 1618, 1518, 1269 cm⁻¹; ¹H-NMR (500 MHz, δ ppm, DMSO-d₆) 11.08 (s, 1H), 10.31 (s, 1H), 9.51 (s, 1H), 8.62 (s, 1H), 8.23 (s, 1H), 7.64 (t, *J* = 5.3 Hz, 1H), 7.61 (s_{b}, 1H), 7.58 (d, *J* = 8.6 Hz, 2H), 6.87 (s, 1H), 6.82 (d, *J* = 8.6 Hz, 2H), 6.63 (d, *J* = 2.5 Hz, 1H), 3.24 (dd, *J* = 12.5 Hz, *J'* = 6.6 Hz, 2H), 1.97 (t, *J* = 7.3 Hz, 2H), 1.56 - 1.49 (m, 4H), 1.33 - 1.27 (m, 2H); ¹³C-NMR (126 MHz, δ ppm, DMSO-d₆) 169.0, 160.7, 156.7, 142.2, 140.8, 133.6, 126.0, 122.7, 121.5, 119.7, 119.5, 115.5, 111.4, 106.2, 38.6, 32.2, 28.9, 26.1, 24.9.

### Example 6: Preparation of N-(5-(Hydroxycarbamoyl)pentyl)-5-(4-hydroxyphenyl)-3-(3-thienyl)-1H-pyrrole-2-carboxamide, with the following structural formula:

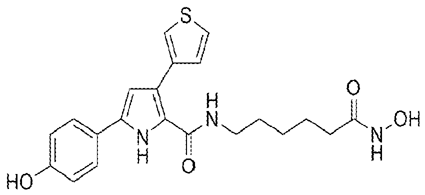

This compound was prepared using a method substantially similar to that of Example 1: Yield 91%; m.p. 85-87°C; IR 3396, 3220, 1641, 1662, 1547, 1281 cm⁻¹; ¹H-NMR (500 MHz, δ ppm, DMSO-d₆) 11.19 (s, 1H), 10.31 (s, 1H), 9.49 (s, 1H), 8.61 (s, 1H), 7.78 (d, *J* = 1.8 Hz, 1H), 7.60 (d, *J* = 8.6 Hz, 2H), 7.49 (dd, *J* = 4.8 Hz, *J'* = 2.9 Hz, 2H), 7.42 (d, *J* = 4.9 Hz, 1H), 6.80 (d, *J* = 8.6 Hz, 2H), 6.59 (d, *J* = 2.3 Hz, 1H), 3.21 (dd, *J* = 12.6 Hz, *J'* = 6.6 Hz, 2H), 1.96 (t, *J* = 7.3 Hz, 2H), 1.54 - 1.45 (m, 4H), 1.30 - 1.24 (m, 2H); ¹³C-NMR (126 MHz, δ ppm, DMSO-d₆) 169.0, 160.9, 156.6, 136.0, 133.3, 129.0, 126.1, 124.7, 122.8, 122.7, 122.0, 121.8, 115.4, 106.7, 38.6, 32.2, 28.8, 26.1, 24.8.

### Biological Activity

Compounds according to the present invention are inhibitors of the histone deacetylases (HDAC). Moreover, the compounds of the present invention are selective HDAC6 inhibitors; in this context "selective" means that they inhibit isotype 6 of the HDAC enzymes at a concentration (IC50) that is at least 20 times lower than that needed for the inhibition of any other HDAC isotype.

### Example 7: In vitro inhibitory activity of histone deacetylase: human isoforms HDAC1, 2, 3, 4, 5, 6, 7, 8, 9, 10 and 11 and HeLa cell line nuclear extract (IC50 data).

### Components of Assay

*Substrate peptides*: All HDAC assays were performed using acetylated AMC-labeled peptide substrate:
- Substrate for isoforms HDAC1, 2, 3, 4, 5, 6, 7, 9, 10, 11 and HeLa nuclear extract assays: Acetylated fluorogenic peptide from p53 residues 379-382 (RHKKAc) (BioMol Cat. # KI-104).
- Substrate for HDAC8 assays: Acetylated fluorogenic peptide from p53 residues 379-382 (RHKAcKAc) (BioMol Cat. # KI-178).

*Assay buffer*: 50 mM Tris-HCl, pH 8.0, 137 mM NaCl, 2.7 mM KCI, 1 mM MgCl₂ (supplement with 1 mg/ml BSA for dilution) (BioMol Cat. # KI-143).

### Enzymes:

- HDAC1 assay: 75 nM Human HDAC1 (GenBank Accession No. NM_004964): Full length with C-terminal GST tag, MW= 79.9 kDa, expressed by baculovirus expression system in Sf9 cells (BioMol Cat. # SE-456).
- HDAC2 assay: 5 nM Human HDAC2 (GenBank Accession No. Q92769): Full length with C-terminal His tag, MW= 60 kDa, expressed by baculovirus expression system in Sf9 cells (BioMol Cat. # SE-500).
- HDAC3 assay: 2.3 nM Human HDAC3/NcoR2 (GenBank Accession No. NM_003883 for HDAC3, GenBank Accession No.NM_006312 for NcoR2): Complex of human HDAC3, full length with C-terminal His tag, MW= 49.7 kDa, and human NCOR2, N-terminal GST tag, MW= 39 kDa, co-expressed in baculovirus expression system (BioMol Cat. # SE-507).
- HDAC4 assay: 266 nM Human HDAC4 (GenBank Accession No. NM_006037): Amino acids 627-1085 with N-terminal GST tag, MW= 75.2 kDa, expressed in baculovirus expression system (BioMol, Hamburg, Germany).
- HDAC5 assay: 588 nM Human HDAC5 (GenBank Accession No. NM_001015053): Full length with Nterminal GST tag, MW= 150 kDa, expressed by baculovirus expression system in Sf9 cells (BioMol, Hamburg, Germany).
- HDAC6 assay: 13 nM Human HDAC6 (GenBank Accession No. BC069243): Full length with N-terminal GST tag, MW= 159 kDa, expressed by baculovirus expression system in Sf9 cells (BioMol Cat. # SE-508).
- HDAC7 assay: 962 nM Human HDAC7 (GenBank Accession No. AY302468): Amino acids 518-end with N-terminal GST tag, MW= 78 kDa, expressed in baculovirus expression system (BioMol, Hamburg, Germany).
- HDAC8 assay: 119 nM Human HDAC8 (GenBank Accession No. NM018486): Full length, MW=42 kDa, expressed in an E. coli expression system (BioMol Cat. # SE-145).
- HDAC9 assay: 986 nM Human HDAC9 (GenBank Accession No. NM178423): Amino acids 604-1066 with C-terminal His tag, MW= 50.7 kDa, expressed in baculovirus expression system (BioMol, Hamburg, Germany).
- HDAC10 assay: 781 nM Human HDAC10 (GenBank Accession No. NM_032019): Amino acids 1-631 with Nterminal GST tag, MW= 96 kDa, expressed by baculovirus expression system in Sf9 cells (BioMol Cat. # SE-559).
- HDAC11 assay: 781 nM Human HDAC11 (GenBank Accession No. NM_BC009676) with N-terminal GST tag, MW= 66 kDa, expressed in baculovirus expression system (BioMol Cat. # SE-560).
- HeLaNuclear Extract assay: 25 ng/µl Nuclear Extract from HeLa Cells: Prepared by high salt extraction of HeLa nuclei (human cervical cancer cell line), this extract is a rich source of HDAC activity (BioMol Cat. # KI-140).

### Assay procedure

50 µM of substrate peptide (see 'substrate peptides' section above) and an optimal concentration of the corresponding enzyme (see 'enzymes' section above) in the assay buffer and 1% final concentration of DMSO were incubated in the presence of gradient concentrations of inhibitors (10-dose IC50 mode with 3-fold serial dilution) at 30°C for 2 h. The reactions were carried out in a 96-well microplate for fluorometry in a 50 µl reaction volume. After the deacetylation reaction, Fluor-de-Lys-Developer (BioMol Cat. # KI-105) was added to each well to digest the deacetylated substrate, thus producing the fluorescent signal. The reaction was allowed to develop for 45 minutes at 30°C with 5% CO₂; then the fluorescent signal was measured with an excitation wavelength at 360 nm and an emission wavelength at 460 nm in a microplate-reading fluorometer (GeminiXS; Molecular Devices, Sunnyvale, CA). A curve of Deacetylated Standard (Biomol, Cat. # KI-142; made from 100 µM with 1:2 dilution and 10-doses, 6 µl) allowed the conversion of fluorescent signal into micromoles of deacetylated product. All experiments were performed in triplicate. IC50 was calculated by fitting the experimental data to a dose-response curve. DMSO was used as negative control; Trichostatin A (Biomol Cat. # GR-309) was used as positive control inhibitor.

Activity of the compounds described within this invention was compared with methoxylated analogues Compound A (IUPAC name: *N*-[6-(hydroxyamino)-6-oxohexyl]-3-(4-methoxyphenyl)-5-phenyl-1H-pyrrole-2-carboxamide, obtained in house) and Compound B (IUPAC name: N-[6-(hydroxyamino)-6-oxohexyl]-5-(4-methoxyphenyl)-3-phenyl-1H-pyrrole-2-carboxamide, obtained in house). Compounds A and B are disclosed in WO 2007/074176.

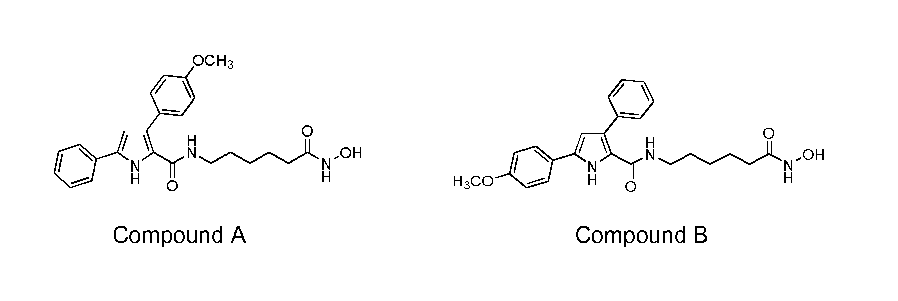

Novel hydroxyphenyl pyrrole derivatives are more potent than the corresponding methoxyphenyl pyrrole derivatives (see Example 1 vs Compound A, and Example 2 vs Compound B in the table 1) inhibiting both HDAC extracts from HeLa cells and isolated HDAC isoforms. In addition, novel hydroxyphenyl pyrrole derivatives are more potent against HDAC6 isoform, and also more selective against this isoform (see table 2) than the corresponding methoxylated analogue:

**Table 1**

| **Compound** | **IC50 (nM) of enzyme activity** | | | | | | | | | | | **IC50 (nM)** |
|---|---|---|---|---|---|---|---|---|---|---|---|---|
| | **HDAC1** | **HDAC2** | **HDAC3** | **HDAC4** | **HDAC5** | **HDAC6** | **HDAC7** | **HDAC8** | **HDAC9** | **HDAC10** | **HDAC11** | **HeLa** |
| **Compound A** | 464 | 1294 | 572 | 1838 | 655 | 20 | 1406 | 192 | 768 | 346 | 680 | 61 |
| **Compound B** | 276 | 918 | 415 | - | - | 32 | - | 174 | - | - | - | 27 |
| **Example 1** | 354 | 1136 | 437 | - | - | 14 | - | 767 | - | - | - | 31 |
| **Example 2** | 153 | 396 | 250 | - | - | 7.6 | - | 162 | - | - | - | 18 |
| **Example 3** | 438 | 1267 | 441 | - | - | 12 | - | 975 | - | - | - | 46 |
| **Example 4** | 353 | 864 | 341 | - | - | 9.3 | - | 591 | - | - | - | 33 |
| **Example 5** | 19 | 56 | 20 | 143 | 14 | 0.6 | 72 | 278 | 27 | 14 | 18 | 1.6 |
| **Example 6** | 49 | 203 | 71 | 499 | 43 | 1.6 | 318 | 104 | 80 | 47 | 55 | 7.3 |

**Table 2**

| **Compound** | **Selectivity against HDAC6 (IC50 HDACx/IC50 HDA56)** | | | | | | | | | |
|---|---|---|---|---|---|---|---|---|---|---|
| | **HDAC1** | **HDAC2** | **HDAC3** | **HDAC4** | **HDAC5** | **HDAC7** | **HDAC8** | **HDAC9** | **HDAC10** | **HDAC11** |
| **Compound A** | 23.2 | 64.7 | 28.6 | 91.9 | 32.7 | 70.3 | 9.6 | 38.4 | 17.3 | 34 |
| **Example 1** | 25.3 | 81.1 | 31.2 | - | - | - | 54.8 | - | - | - |
| | | | | | | | | | | |
| **Compound B** | 8.6 | 28.7 | 13.0 | - | - | - | 5.4 | - | - | - |
| **Example 2** | 20.1 | 52.1 | 32.9 | - | - | - | 21.3 | - | - | - |

## Claims

1. Compound of general formula (I), 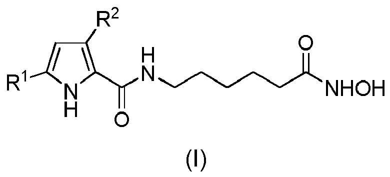 wherein:
R¹ and R² are independently selected between an optionally substituted C₆-C₁₀ aryl and an optionally substituted 3- to 10-membered heteroaryl group, being at least one of R¹ and R² a phenol group;
or a salt, solvate or prodrug thereof.

2. Compound according to claim 1, wherein one of the R¹ and R² represents a phenol group, and the other one represents an optionally substituted phenyl group or an optionally substituted 5- or 6-membered heteroaryl group.

3. Compound according to claim 2, wherein one of the R¹ and R² represents a phenol group, and the other one represents an optionally substituted phenyl group, an optionally substituted furan or an optionally substituted thiophene.

4. Compound of general formula (I) according to any of the preceding claims selected from:
[1] *N*-[6-(hydroxyamino)-6-oxohexyl]-3-(4-hydroxyphenyl)-5-phenyl-1*H*-pyrrole-2-carboxamide, with the following structural formula: 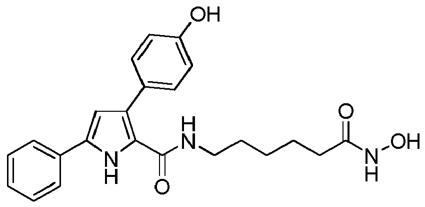
[2] *N*-[6-(hydroxyamino)-6-oxohexyl]-5-(4-hydroxyphenyl)-3-phenyl-1*H*-pyrrole-2-carboxamide, with the following structural formula: 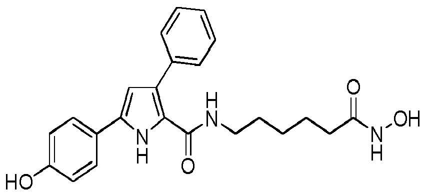
[3] *N*-[5-(Hydroxycarbamoyl)pentyl]-3-(4-hydroxyphenyl)-5-(3-thienyl)-1*H*-pyrrole-2-carboxamide, with the following structural formula: 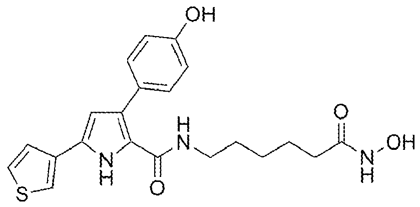
[4] 5-(3-Furyl)-*N*-[5-(Hydroxycarbamoyl)pentyl]-3-(4-hydroxyphenyl)-1*H*-pyrrole-2-carboxamide, with the following structural formula: 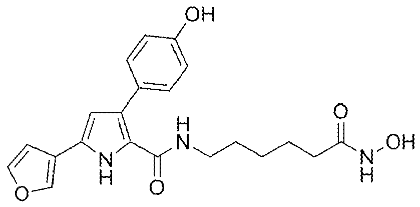
[5] 3-(3-Furyl)-*N*-[5-(hydroxycarbamoyl)pentyl]-5-(4-hydroxyphenyl)-1*H*-pyrrole-2-carboxamide, with the following structural formula: 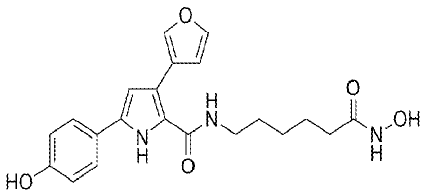
[6] *N*-[5-(Hydroxycarbamoyl)pentyl]-5-(4-hydroxyphenyl)-3-(3-thienyl)-1*H*-pyrrole-2-carboxamide, with the following structural formula: 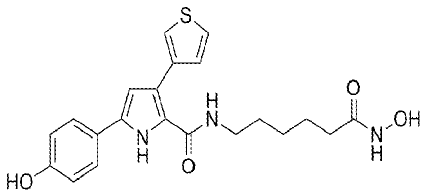 or a salt, solvate or prodrug thereof.

5. Process for the preparation of a compound of general formula (I): 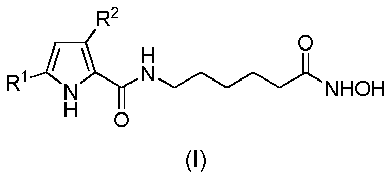 or a salt, solvate or prodrug thereof, wherein R¹ and R² have the meaning given in claim 1, which comprises reacting a mixture of:
a) a compound of formula (II); 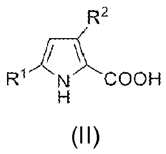
b) a compound of formula (III) or a salt thereof,
H₂N-(CH₂)₅-R³ (III)
wherein R³ is an alkoxycarbonyl;
c) at least one reagent for the activation of the carboxyl group; and
d) at least one tertiary amine;
and reacting the obtained product with a mixture of hydroxylamine hydrochloride and phenolphthalein in the presence of an excess of sodium methoxide in methanol.

6. Process for the preparation of a compound of general formula (I), or a salt, solvate or prodrug thereof, as defined in any of claims 1-4, which comprises reacting a mixture of:
a) a compound of formula (IIP) 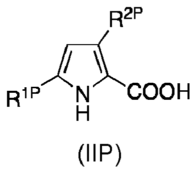 wherein one of the R^{1P} and R^{2P} represents a protected hydroxyphenyl radical, preferably a methoxyphenyl group or a benzyloxyphenyl group, and the other one represents an optionally substituted C₆-C₁₀ aryl or an optionally substituted 3-to 10-membered heteroaryl group;
b) a compound of formula (III) or a salt thereof,
H₂N-(CH₂)₅-R³ (III)
wherein R³ is an alkoxycarbonyl;
c) at least one reagent for the activation of the carboxyl group; and
d) at least one tertiary amine,
performing a deprotection reaction to remove the protecting group of the hydroxyphenyl substituent, and reacting the obtained product with a mixture of hydroxylamine hydrochloride and phenolphthalein in the presence of an excess of sodium methoxide in methanol.

7. Process according to claim 5, wherein the preparation of the compound of general formula (II) comprises:
a) reacting a α,β-unsaturated carbonylic compound with the following formula (IV): 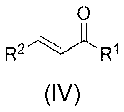 an ester of the nitroacetic acid of general formula (V): 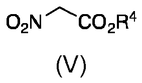 where R⁴ is a C₁-C₆ alkyl group; and a primary, secondary or tertiary amine or an inorganic base, to obtain a compound of general formula (VI): 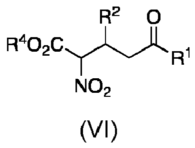
b) subjecting the mentioned compound of formula (VI) to an oxidation reaction to yield a compound of general formula (VII): 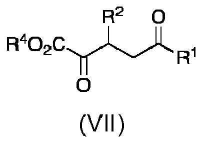
c) treating the mentioned esters of general formula (VII) with ammonium hydroxide or an ammonium salt of an aliphatic carboxylic acid of less than 5 carbon atoms, and subsequent alkaline hydrolysis.

8. Process according to claim 6, wherein the preparation of the compound of general formula (IIP) comprises:
a) reacting a mixture formed by a nitroalkene of configuration E or Z with the following formula (VIII),
O₂N-CH=CH-R^{2P} (VIII)
wherein R^{2P} represents a protected hydroxyphenyl radical, an optionally substituted C₆-C₁₀ aryl or an optionally substituted 3-to 10-membered heteroaryl group;
an imine of configuration E or Z with the following formula (IX),
R^{1P}-CH=N-CH₂-COOR⁵ (IX)
wherein R^{1P} represents a protected hydroxyphenyl radical, an optionally substituted C₆-C₁₀ aryl or an optionally substituted 3-to 10-membered heteroaryl group and R⁵ represents a C₁-C₆ alkyl or C₆-C₁₀ aryl group;
a metal salt and a tertiary organic amine in an organic solvent and at a temperature between -25°C to +25°C or alternatively using microwave irradiation;
b) treating the obtained 2-alkoxycarbonyl pyrrolidine isomers with an oxidizing agent and thermal heating or microwave irradiation; and
c) subjecting the obtained 2-alkoxycarbonyl 1*H*-pyrrole to alkaline hydrolysis.

9. Compound of formula (I) according to any of claims 1 to 4, or a pharmaceutically acceptable salt, solvate or prodrug thereof, for use as a medicament.

10. Compound of formula (I) according to any of claims 1 to 4, or a pharmaceutically acceptable salt, solvate or prodrug thereof, for use as a medicament for the treatment of cancer.

11. Compound of formula (I) according to any of claims 1 to 4, or a pharmaceutically acceptable salt, solvate or prodrug thereof, for the treatment and/or prevention of diseases or disorders responsive or sensitive to the inhibition of histone deacetylases.

12. Compound of formula (I) according to claim 11, wherein the diseases or disorders responsive or sensitive to the inhibition of histone deacetylases are selected from proliferative diseases; hematological malignancies; autoimmune diseases; inflammatory diseases; cardiovascular diseases; diseases of the central nervous system (CNS); psychiatric disorders; and endocrine and metabolic disorders

13. Compound of formula (I) according to claim 12, wherein
- the proliferative diseases are selected from cancer, benign prostatic hyperplasia, endometriosis and oral leukoplakia;
- the inflammatory diseases are selected from skin inflammatory diseases such as psoriasis, acne or eczema; musculoskeletal inflammatory diseases such as rheumatoid arthritis, juvenile rheumatoid arthritis, ankylosing spondylitis or osteoarthritis; inflammatory conditions of the gastrointestinal tract such as inflammatory bowel disease, Crohn's disease, ulcerative colitis or irritable bowel syndrome);
- the cardiovascular diseases are selected from stroke, myocardial infarction, cardiac hypertrophy and chronic heart failure;
- the diseases of the central nervous system (CNS) are neurodegenerative diseases such as Huntington's disease, Rubinstein-Taybi syndrome or Rett syndrome;
- the psychiatric disorders are selected from schizophrenia and anxiety; and
- the endocrine and metabolic disorders is diabetes.

14. Pharmaceutical composition which comprises at least a compound of formula (I) according to any of claims 1 to 4, or a pharmaceutically acceptable salt, solvate or prodrug thereof, and a pharmaceutically acceptable excipient.
